# EUROPEAN PATENT SPECIFICATION

(11) **EP 4 027 935 B1**
(45) Date of publication and mention of the grant of the patent: **20.08.2025**
(21) Application number: 20864245.4
(22) Date of filing: 14.09.2020
(51) Int. Cl.: A61C 7/08, A61F 5/56, A61M 16/00, A61C 3/00

(54) **LINGUAL POSITIONING DEVICE FOR SLEEP, BREATHING, JAW AND AIRWAY DISORDERS**
LINGUALE POSITIONIERVORRICHTUNG FÜR SCHLAF-, ATMUNGS-, KIEFER- UND ATEMWEGSERKRANKUNGEN
DISPOSITIF DE POSITIONNEMENT LINGUAL POUR TROUBLES DU SOMMEIL, DE LA RESPIRATION, DE LA MÂCHOIRE ET DES VOIES RESPIRATOIRES

(30) Priority: 12.09.2019 US 201962899467 P; 17.09.2019 US 201962901589 P; 18.10.2019 US 201962923182 P; 18.10.2019 US 201962923199 P; 18.10.2019 US 201962923208 P; 25.06.2020 US 202063044235 P
(43) Date of publication of application: 20.07.2022
(73) Proprietor: My LIttle Projects Series 8 Planco 25 LLC, Georgetown, TX 78626 (US)
(72) Inventor: PUMPHREY, Willis, J., Georgetown, TX 78626 (US)
(74) Representative: WP Thompson
(86) International application number: PCT/US2020/050786
(87) International publication number: WO 2021/051112

(56) References cited:
- EP-B1- 2 693 995
- US-A1- 2010 311 008
- US-A1- 2018 000 631
- US-A1- 2018 000 631
- US-B1- 8 881 733
- US-B2- 8 833 374

## Description

### BACKGROUND OF THE INVENTION

### 1. Field of the Invention.

The present invention relates to oral appliances.

### 2. Description of Related Art.

A malocclusion is a misalignment or incorrect relation between the teeth of the two dental arches when they approach each other as the jaws close. Orthodontic treatment/appliances are utilized for the prevention and correction of malpositioned teeth and jaws.

For example, crowding of the teeth is treated with orthodontics, often with tooth extraction, clear aligners, or dental braces, followed by growth modification in children or jaw surgery (orthognathic surgery) in adults. Surgery may be required on rare occasions. This may include surgical reshaping to lengthen or shorten the jaw (orthognathic surgery). Wires, plates, or screws may be used to secure the jawbone, in a manner like the surgical stabilization of jaw fractures.

While clear aligners are quite commonly utilized in orthodontic treatment, *[e]xperience suggests they are effective for moderate crowding of the front teeth, but less effective than conventional braces for several other issues and are not recommended for children. In particular they are indicated for "mild to moderate crowding (1-6 mm) and mild to moderate spacing (1-6 mm)", in cases where there are no discrepancies of the jawbone.* See, Wikipedia, https://en.wikipedia.org/wiki/Clear_aligners, August, 27, 2019, 10:52CST.

Various types of oral appliances are proposed for various sleep and breathing disorders ("SBD"), sleep apnea, airway obstruction, airway reshaping, mandibular advancement, and tongue-retaining.

Airway disorders affect a large portion of the population and are regarded as multifactorial conditions with a multitude of etiologies and treatment modalities. Airway disorders affect a significant population with estimates of the 3% of pediatric population and 10% of the adult population. Morbidity from airway disorders ranges from mental and physical deterioration to disabling illnesses or even death. In children, physical and cognitive development can be delayed or stunted. Adult manifestations can include tiredness, somnolence, memory loss and sleep disorders.

Obstructive sleep apnea (OSA) is a major health risk that various oral and dental appliances have been addressing for years. However, these appliances merely address the symptoms associated with OSA-they are not therapeutic in nature.

It is easy to be dismissive of the damage done by sleep apnea.

However, sleep apnea is not merely a condition of snoring or loud breathing during sleep, and thus is more than an annoyance to those sleeping nearby. The term apnea refers to the momentary (10 seconds or more in duration is considered an apnea) cessation of breathing and sleep apnea is a momentary cessation of breathing during sleep. Severe sleep apnea can deprive the sleeper of sufficient oxygen for a restful sleep, and the sudden cessation will usually cause the sleeper to awake. Several episodes a night will deprive the sleeper of sufficient sleep to function alertly the next day. Yet, such sleep apnea is generally a chronic condition, and it can be difficult to find a solution in many cases.

Sleep apnea may have various causes, such as the momentary blockage of the windpipe by the tongue, which may occur particularly when the sleeper is already breathing through his her mouth. While this may or may not be fatal, it nevertheless creates an apneic episode and interrupts sound sleep. As a result, a number of devices have been developed in the past to treat such episodes of sleep apnea, ranging from mechanical devices intended for placement in the mouth to restrain movement of the tongue to more complex and costly devices, such as Continuous Positive Airway Pressure machines (CPAP, APAP or BPAP) and other electronic devices. The more costly and complex electronic devices are beyond the means of many people who can benefit from such devices. Research has showed that patients have more tolerance and use for oral appliance than for CPAP. Furthermore, Oral appliance yields better results than surgical intervention.

Obstructive sleep apnea occurs when the muscles (of the airway) in the back of the throat relax. These muscles support the soft palate, the triangular piece of tissue hanging from the soft palate (uvula), the tonsils, the side walls of the throat and the tongue. When the muscles relax, the airway narrows or closes as one breathes in, and cannot get an adequate breath. This will eventually lower the level of oxygen in one's blood.

Brain senses this inability to breathe and briefly wakes the person up so that she can reopen her airway. This awakening is usually so brief that the person, in most cases, does not remember it. A person with sleep apnea may make a snorting, choking or gasping sound. This pattern can repeat itself five to 30 times or more each hour, all night long. The highest apneic episodes occurs during supine positions and in REM stage. These disruptions impair one's ability to reach the desired deep, restful phases of sleep, and she will probably feel sleepy during the waking hours. People with obstructive sleep apnea may not be aware that their sleep was interrupted. In fact, some people with this type of sleep apnea think they sleep well all night.

Oral appliances that are used to alleviate snoring and obstructive sleep apnea are devices worn in the mouth, similar to orthodontic retainers or sports mouthguards. They have several advantages over other forms of therapy. Oral appliances are relatively inexpensive, easy to travel with, non-invasive, easy to fabricate, and quite well accepted by patients. The appliance works non-invasively to assist in keeping the airway open and its effectiveness has been superior to when it is compared with various invasive surgical interventions for the treatment of Obstructive Sleep Apnea (OSA).

Oral appliance therapy is becoming recognized by the dental and medical community as an effective treatment option in the management of (obstructive) sleep breathing disorders. Medical and dental researchers have joined together to evaluate and improve the outcome of the use of oral appliances. It is generally recommended that oral appliances be used in patients with primary snoring or mild (to moderate) obstructive sleep apnea and inpatients who are intolerant of or refuse treatment with continuous positive airway pressure devices (CPAP).

Oral appliances fall into two basic categories: tongue-retaining devices (TRD) and mandibular advancing devices (MAD). Tongue-retaining devices are small acrylic (silicone) devices which fit by suction on the tip (anterior portion) of the tongue by various controlled and measurable mechanical means. Mandibular advancing devices are small acrylic devices that fit over the upper and lower teeth and advance the lower jaw as well as increase the vertical opening of patient's mouth in order to facilitate airway patency during various stages of sleep. In addition, it facilitates advancing of the mandible. Both move the base of the tongue forward and open the airway. In general, TRDs do not work as well as MADs. They are non-invasive, easy to use and are effective in improving breathing, reducing snoring and reducing sleep apnea. They are less effective for treating people with moderate or severe obstructive sleep apnea and people who are obese. MAD may be used in combination with CPAP due to better compliance and in order to keep the airway patent when patient fails to use CPAP; particularly, with patients that fail to utilize their CPAP on regular basis as prescribed for them.

TRD's are typically custom-made appliances with an anterior bulb that holds the tongue in a forward position during sleep by means of negative pressure (or suction). Currently, the TRD appears to be the appliance of choice for patients who have few or no teeth and for patients who have large tongues. In addition, the TRD is a good appliance for patients who cannot adequately advance their lower jaw for whatever reason. Its disadvantages are that it is more difficult for both clinicians and patients to use on a regular basis, it poses problems for patients who cannot breathe through their nose, it irritates the end of the tongue over time and it is a single jaw and tongue position appliance.

MAD devices fit over both the upper and the lower teeth and move the lower jaw forward, which brings the tongue and some throat tissues along with it in order to prevent collapsing the airway. They also stimulate the pharyngeal muscles to maintain an open airway during REM sleep-the muscles that normally relax during various stages of sleep, including REM. There are several different designs for MADs. Some designs are not adjustable after fabrication and have to be remade if any alterations are required. If the devise if not adjustable, it cannot pass the FDA requirement for treatment of OSA and therefore cannot be used. Others have an excellent range of adjustment. MAD appliances, in general, require a healthy jaw joint (TMJ) and several healthy teeth with healthy gums in both the upper and lower jaw.

The prior art is replete with many examples of oral appliances, the following of which are merely a sampling.

U.S. Patent No. 7,314,372, issued January 1, 2008, to Belfor et al., discloses a method and apparatus are provided for changing the form of the jaw and facial bones of an adult patient that did not develop fully during childhood. The method utilizes a device having a plate body with an expansion screw device that fits within the mouth of the patient, flap springs that project from the plate body and an overlay extending from the plate body. The device is placed within the mouth of the patient so that the overlay is in a position between at least two opposing teeth. In this position the flap springs press against selected teeth that are out of alignment in order to urge those teeth back into place. This force on the teeth causes the jawbone to expand to accept the teeth in their proper position(s). Also, the device is arranged such that the opposing teeth contact the overlay during swallowing, which causes the patient's facial muscles to intermittently pull on the facial bones.

U.S. Patent No. 7,357,635, issued April 15, 2008, to Belfor et al., discloses a method and apparatus for changing the form of the jaw and facial bones of an adult patient that did not develop fully during childhood. The method utilizes a device having a plate body with an expansion screw that fits within the mouth of the patient; flap springs that project from the plate body, and an overlay extending from the plate body. The device is placed within the mouth of the patient so that the overlay is in a position between at least two opposing teeth. In this position, opposing teeth contact the overlay during function (e.g. swallowing). This intermittent, unilateral application of force to the facial bones causes these bones to further develop, positioning out of place teeth into more proper positions, and inducing a more symmetrical and enhanced appearance of the face, as well as increasing the airway space behind the jaws. Concomitantly, the flap springs gently press against selected teeth that are out of alignment in order to guide those teeth into place.

U.S. Patent No. 7,559,328, issued July 14, 2009, to Eubank, discloses an oral appliance for maintaining stability of one or more aspects of a user's masticatory system includes a first arch adapted to receive at least some of the user's teeth and a second arch adapted to receive at least some of the user's teeth. The first arch includes an anterior substantially planar region located proximate a midline of the first arch. The second arch includes an anterior bearing platform located proximate a midline of the second arch, elongated in an anterior-posterior direction, and operable to contact the anterior substantially planar region of the first arch when the user bites down with the oral appliance inserted in the user's mouth and the user's temporomandibular joint in its proper natural position to help maintain stability of one or more aspects of the user's masticatory system.

U.S. Patent No. 7,637,262, issued December 29, 2009, to Bailey, discloses an oral appliance for treating sleep apnea and bruxism is formed of a maxillary tray and a mandibular tray. The maxillary tray carries a maxillary bite pad projecting downwardly toward an opposed mandibular occlusal surface. The mandibular tray carries a mandibular bite pad projecting upwardly toward an opposed maxillary occlusal surface. The maxillary bite pad is posterior to the mandibular bite pad such that upon sufficient relative posterior movement of the mandibular tray, the maxillary bite pad engages the mandibular bite pad and limits posterior movement of the mandibular tray. At least one surface, chosen from the anterior face of the maxillary bite pad and the posterior face of the mandibular bite pad, defines a guide plane disposed at an upward and forward slant. The maxillary bite pad and the mandibular bite pad are engageable at the guide plane for advancing the mandibular tray along the guide plane upon vertical closure between the trays. A smooth pad is disposed over the occlusal surface of at least one of the trays, guarding the occlusal surface from engagement with the opposed bite pad.

U.S. Patent No. 7,794,399, issued September 14, 2010, to Singh, discloses a system and method for three-dimensional airway reconstruction, assessment and analysis. Specifically, the invention relates to a system and method for acquiring one- and two-dimensional data regarding a cavity, such as an esophagus or an airway, and manipulating that data to reconstruct a three-dimensional geometrical object representing that cavity. Suitable data collection methods include, but are not limited to, non-ionizing, non-invasive protocols including acoustic reflectometry, such as that performed by a DOS^{®}- or Windows^{®}-based pharyngometer or rhinometer. The resulting three-dimensional geometric object of the subject cavity can be used to diagnose cavity morphology/obstruction, aid in management and treatment of the obstruction, evaluate efficacy of management and treatment of the obstruction and also provide information for use in outcome analysis and forensic and medico-legal evaluation of diagnosis and treatment of cavity obstruction/stenosis.

U.S. Patent No. 7,887,324, issued February 15, 2011, to Singh, discloses an osteogeneticorthodontic appliance, device, system, and method optimizes craniofacial homeostasis by means of a 3-D axial spring that influences the patient's genome and thereby addresses problems existing primarily within the mid-facial region, as well as the other contiguous regions. Growth and development of the craniofacial structures can be influenced by foundational (skeleto-dental) correction in concert with functional (myo-spatial) correction, according to the genome of a particular patient by means of the method and systems of the present invention.

U.S. Patent Publication No. 20110066058, published March 17, 2011, by Singh, discloses a system and method for three-dimensional airway reconstruction, assessment and analysis. Specifically, the invention relates to a system and method for acquiring one- and two-dimensional data regarding a cavity, such as an esophagus or an airway, and manipulating that data to reconstruct a three-dimensional geometrical object representing that cavity. Suitable data collection methods include, but are not limited to, non-ionizing, non-invasive protocols including acoustic reflectometry, such as that performed by a DOS^{®}- or Windows^{®}-based pharyngometer or rhinometer. The resulting three-dimensional geometric object of the subject cavity can be used to diagnose cavity morphology/obstruction, aid in management and treatment of the obstruction, evaluate efficacy of management and treatment of the obstruction and also provide information for use in outcome analysis and forensic and medico-legal evaluation of diagnosis and treatment of cavity obstruction/stenosis.

U.S. Patent Publication No. 20110269091, published November 3, 2011, by Li et al, and now issued as U.S. Patent No. 9,241,774, discloses a patterned dental positioning appliance. One method embodiment includes receiving a dental mold of a patient's dentition, applying a pattern to a portion of the dental mold, and applying a material to the dental mold to form the removable dental positioning appliance that includes either the pattern on the portion of the dental mold or an inverse thereof.

U.S. Patent No. 8,192,196, issued June 5, 2012, to Singh, discloses a non-rigid, epigenetic-pneumopedic appliance comprising a wire-type framework supporting bow, bands, brackets or a clasp. The appliance includes active elements adapted to provide brief doses of cyclic forces to induce sutural osteogenesis. The active elements are vibrational, ultrasonic or oscillatory components. The epigenetic-pneumopedic appliance cooperates with an actuator or other expansion mechanism, such as suture spring, that straddles the midline of the appliance. The appliance further includes tooth-contacting material having high-elasticity, such as pre-formed alloys that form 3-D axial springs, which adapt to the long axis of the palatal/lingual surfaces of the teeth. The appliance also includes a plurality of micro-screws 2 along with driving means preferably consisting of either an electrical, ultrasonic (vibrational) meso-motor, or, alternatively, a micro-motor. The epigenetic-pneumopedic appliance may be used with directional bite props.

U.S. Patent Publication No. 2016/0256240, published September 8, 2016, by Shivapuja et al., discloses direct 3D-printed orthodontic aligners with torque, rotation, and full-control anchors are provided. In an implementation, an orthodontic system uses 3D-printed aligners to perform orthodontic treatment of teeth. Manufacturing the aligners by 3D-printing or additive manufacturing processes imparts novel geometries to the aligners for applying torque, rotation, and full 3D control forces to the teeth in novel ways. The 3D-printed aligners may contain multiple different plastic or metal materials with different orthodontic properties. In an implementation, divot anchors can be strategically applied to teeth to work in conjunction with a 3D-printed aligner fitted over the divot anchors and teeth. The divot anchors may include a depression, channel, groove, or notch providing an attachment point for the aligner to apply a force to the tooth through the divot anchor, such as a torque, rotational force, leverage, push, pull, or 3D control force.

U.S. Patent Publication 2017/0007364 published January 12, 2017, by Wu, discloses a dual aligner assembly including a plurality of aligners, including a first aligner and a second aligner. The first aligner has a first shape corresponding to a set of target tooth positions and applies an orthodontic force against a set of target teeth. The first orthodontic force generates movement of the set of target teeth to the set of target tooth positions. The second aligner has a second shape corresponding to a combination of current tooth positions of the set of target teeth, the set of target tooth positions, and a thickness of the first aligner. The second aligner partially encloses the first aligner and provides an anchor for at least a portion of the first aligner. Via the anchor, a combination of the first aligner and the second aligner provides an orthodontic force that prevents the set of target teeth from moving to unwanted tooth positions during orthodontic treatment.

U.S. Patent Publication No. 2017/0156821, published June 8, 2017 by Kopelman et al, and now issued as U.S. Patent No. 9,795,461, discloses method, systems, and devices for expanding arch of teeth. A device for expanding an arch of teeth of a patient can include a removable shell formed of a first material having a number of cavities formed therein. The number of cavities are shaped to receive teeth of a patient. A device for expanding an arch of teeth of a patient can include an arch element extending from the removable shell in a lingual direction and across an arch width of the removable shell. The arch element can be formed of the first material and a second material that is a different material than the first material, can be designed to expand an arch of the teeth of the patient, and can have a width specific to a stage of a treatment plan.

U.S. Patent Publication No. 2017/0304108, published October 26, 2017, by Simonetti, discloses an orthotic device for redefining an occlusal plane includes a left portion, a right portion including an upper surface, a bite block coupled to either the left portion or the right portion, and a lingual portion coupled to the left portion and the right portion. The orthotic device is configured to engage teeth located along a left portion and a right portion of a mandible. The bite block is disposed along the upper surface of either the left portion or the right portion and extends upward relative to an upper surface of the left or right portion.

U.S. Patent Publication No. 2018/0000563, published January 4, 2018, by Shanjani et al., discloses detection of placement of dental aligners in patient mouth on teeth for indication of wearing compliance. Described herein are apparatuses and methods for detecting wearing, including compliance, and for reliably transferring data, by wired or wireless direct or indirect communication of electronic compliance information to a smartphone. Also described herein are dental appliances that can detect physiological parameters related to respiration and sleep. Also described herein are aligner cases that enable NFC communication with electronic compliance indicator (ECI) devices and Bluetooth communication with smartphones.

U.S. Patent Publication No. 2018/0071130, published March 15, 2018, by Simonetti et al., discloses methods for stimulation of craniofacial osteogenesis which includes the step of providing light intermittent cyclic tensile force to the craniofacial area of a patient. In an embodiment, the step of providing mechanical stress is achieved through the use of removable oral orthotic devices which utilize unilateral bite block technology.

U.S. Patent Publication No. 2018/0092713, published April 5, 2018, by Boehlau et al., discloses a multiple-stage treatment method of orthodontically correcting teeth of a patient. The patient may wear, during the night and whenever convenient, a tooth positioner shaped to fit their dentition. The patient may then wear, at other times, an arch aligner shaped to fit their dentition. The patient may progress in stages, with the patient using a different arch aligner at every stage and a different tooth positioner every several stages. The combination of the arch aligner and tooth positioner may be used to treat, among other problems, class II and class III malocclusions, or transverse or vertical malocclusions, while maximizing the potential for patient compliance and reducing the number of tooth positioners and/or arch aligners that have to be made.

U.S. Patent Publication No. 2019/0105829, published April 11, 2019, by Sato et al., discloses method, computing device readable medium, and devices for dental appliances formed with folded material components. An example of a method of forming a dental appliance, includes forming a shell having a number of tooth apertures configured to receive and reposition a number of teeth of a patient along one jaw of a patient, the shell having a number of specialized components and wherein the number of specialized components are formed from folding multiple sections of the first sheet of material over each other.

U.S. Patent Publication No. 2019/0159870, published May 30, 2019, by Li et al., discloses a dental appliance for positioning a patient's teeth includes a removable orthodontic tooth positioning appliance having teeth receiving cavities shaped to directly receive at least some of the patient's teeth and apply a resilient positioning force to the patient's teeth. The appliance includes a hard polymer layer having a hard polymer layer elastic modulus disposed between a first soft polymer layer having a first soft polymer layer elastic modulus and a second soft polymer layer having a second soft polymer layer elastic modulus. The hard polymer layer elastic modulus is greater than each of the first soft polymer layer elastic modulus and the second soft polymer layer elastic modulus. At least one of the first soft polymer layer and the second soft polymer layer has a flexural modulus of greater than about 35,000 psi.

U.S. Patent Publication No. 2019/0167113, published June 6, 2019, by Kopelman et al., discloses systems and methods are provided for preparation of orthodontics and prosthodontics. A method may include scanning a patient's teeth to form first 3D data of the patient's teeth including a removable element that obscures part of the dental surfaces of the patient's teeth and nonobscured tooth surfaces, removing the removable element form the patient's teeth so that the removable element no longer obscures the part of the dental surfaces of the patient's teeth, and scanning the previously obscured part of the dental surfaces of the patent's teeth and the nonobscured tooth surfaces.

U.S. Patent Publication No 2019/0231477, published August 1, 2019, by Shanjani et al., discloses sensors for monitoring an oral appliance (such as an aligner, palatal expander, mandibular repositioning device, etc.) to determine if the oral appliance is functioning properly and/or if it has developed a defect.

U.S. Patent Publication No. 2019/0231482, published August 1, 2019, by Moon et al., discloses an orthodontic framework and methods of making and using the same. The framework is configured to be placed within a dental arch nearby a tooth or teeth of a patient in combination with an appliance via an attachment for moving the tooth or teeth, the framework being less pliable than the appliance so as to serve as an anchor or a backbone of the appliance, the appliance is effective to move a tooth or teeth from an initial position to an intermediate position or final position of the tooth or teeth according to a prescription by a treating doctor, and the attachment is via a material forming the appliance and is configured to allow the framework to exert a non-reciprocal force upon the appliance and the appliance to exert a non-reciprocal force upon the tooth or teeth.

Prior art document US 2018/000631 A1 discloses a tongue grasping and restraining device holds the tongue securely during sleep in order to minimize the risk of the tongue slipping back and blocking the throat, a condition known as obstructive sleep apnea. Plural tongue gripping projections are coupled to upper and lower supports. The projections engage and hold the tongue. One or more of the upper and lower supports are biased together into a tongue engaging position. A tongue depressor can extend rearwardly and downwardly from the upper support to depress the user's tongue to further open the user's airway. The position of the tongue depressor can be adjustable. The upper support is coupled to the user's upper jaw, for example, to a denture, dental appliance or other form of an upper jaw coupler and can be pivoted thereto. The lower support can be hinged to, fixed to, or otherwise joined to a lower jaw coupler. A tube and rod mechanism in one embodiment couples the upper and lower jaw couplers together. US 2018/000631 A1 discloses the preamble of independent claim 1.

However, in spite of the many advancements in the prior art, there remains a need for improved oral appliances, to parts thereof, to systems thereof, and to methods of making and using such parts, appliances, and systems.

There also remains a need for improved orthodontic appliances, to parts thereof, to systems thereof, and to methods of making and using such parts, appliances and systems.

There even also remains a need for improved appliances for reshaping airways, to parts thereof, to systems thereof, to methods of making such parts, appliances and systems, and to method of reshaping airways using such parts, appliances and systems.

There still also remains a need for improved appliances, to parts thereof, and to systems thereof for both orthodontic treatment and reshaping airways, to methods of making such parts, appliances and systems, and to methods of both orthodontic treatment and shaping/reshaping airways using such parts, appliances and systems.

There yet also remains a need for improved appliances for sleep and breathing disorders, to parts thereof, to systems thereof, to methods of treating sleep and breathing disorders ("SBD"), and to methods of making and using such parts, appliances and systems.

There even still also remains a need for improved sleep and breathing disorder systems generally comprising a series of aligners used in conjunction with a series of SBD appliances, and to methods of making a using such systems.

There even yet also remains a need for aligner systems that can be utilized for more than just mild to moderate crowding of the front teeth, for more than just for mild to moderate spacing, for children, and/or for cases in which discrepancies of the jawbone are present.

The problem with many jaw expansion devices/systems is that during the jaw expansion phase, the teeth are allowed to drift into what may be non-desirable positions. Thus, at the end of jaw expansion, there is usually the need for repositioning of one or more teeth, meaning that orthodontic methods/systems are then applied at the end of jaw expansion. Thus, there still even also remains a need in the art for jaw expansion methods and equipment that will not only provide for jaw expansion, but at the same time move the teeth into or toward their desired position.

These and other needs will become apparent to those of skill in the part upon review of this application.

### BRIEF SUMMARY OF THE INVENTION

Some non-limiting embodiments of the present invention provide for improved oral appliances are disclosed in the dependent claims.

### BRIEF DESCRIPTION OF THE DRAWINGS

The following drawings illustrate some of the many possible embodiments of this disclosure in order to provide a basic understanding of this disclosure. These drawings do not provide an extensive overview of all embodiments of this disclosure. These drawings are not intended to identify key or critical elements of the disclosure or to delineate or otherwise limit the scope of the claims. The following drawings merely present some concepts of the disclosure in a general form. Thus, for a detailed understanding of this disclosure, reference should be made to the following detailed description, taken in conjunction with the accompanying drawings, in which like elements have been given like numerals.
FIG. 1A is an isometric view of one non-limiting embodiment of tongue guiding apparatus 10 of the present invention positioned on a lower set of teeth 18 comprising one or more teeth 15, with this tongue guiding apparatus 10 comprises a supporting shell member 12, and a tongue guide member 14.
FIG. 1B is a top view of one non-limiting embodiment of tongue guiding apparatus 10 of the present invention positioned on a lower set of teeth 18 comprising one or more teeth 15, with this tongue guiding apparatus 10 comprises a supporting shell member 12, and a tongue guide member 14.
FIG. 1C is a cutaway view from FIG. 1B, and shows a cross-sectional view of one non-limiting embodiment of tongue guiding apparatus 10 of the present invention positioned on a lower set of teeth 18 comprising one or more teeth 15, with this tongue guiding apparatus 10 comprises a supporting shell member 12, and a tongue guide member 14.
FIG. 2A shows tongue guide 14 extending from the front teeth (i.e., incisors 15A and others) back toward but not impinging upon frenulum, with an edge 14E facing the frenulum that is concave relative to the frenulum.
FIG. 2B shows tongue guide 14 extending from extends from the front teeth (i.e., incisors 15A and others) back toward and impinging upon frenulum F, with an edge 14E facing the frenulum that is convex relative to the frenulum.
FIG. 2C shows tongue guide 14 extending from extends from the front teeth (i.e., incisors 15A and others) back toward frenulum F, with a frenulum receiving zone 14B defined between tongue guide extensions 14A (which do not extend past frenulum F), with the edge facing the frenulum having both concave and convex portions relative to the frenulum.
FIG. 2D shows tongue guide 14 extending across the premolars and/or molars, not in contact with the front incisors 15A, and not impinging upon frenulum F.
FIG. 2E shows tongue guide 14 extending from the front incisors 15A and back toward but not impinging upon frenulum F.
FIG. 2F shows tongue guide 14 extending from extends from the front teeth (i.e., incisors 15A and others) back toward frenulum F, with a frenulum receiving zone 14B defined between tongue guide extensions 14A (which do not extend past frenulum F), with the edge facing the frenulum presenting a flat face relative to the frenulum.
FIGs. 2G, 2H, and 2I, multiple tongue guides 14 extend from the lingual side of the teeth back toward but not impinging upon frenulum F (which do not extend past frenulum F). It should be understood that any suitable number of tongue guides may be employed for use in the present invention.
FIG. 3A shows tongue guide 14 comprising extensions 14A which extend past each side of frenulum F to define a frenulum accommodation zone 14B for receiving frenulum F.
FIG. 3B shows tongue guide 14 comprising extensions 14A which extend past each side of frenulum F to define a frenulum accommodation zone 14B for receiving frenulum F, with impingement upon frenulum F.
FIG. 3C shows tongue guide 14 only extending from the front teeth (incisors 15A), and comprises extensions 14A which extend past each side of frenulum F to define a frenulum accommodation zone 14B for receiving frenulum F.
FIG. 3D shows tongue guide 14 extending across the lower teeth 18, and comprises extensions 14A which extend past each side of frenulum F to define a frenulum accommodation zone 14B for receiving frenulum F.
FIG. 3E shows tongue guide 14 having extensions 14A which extend past each side of frenulum F to define a frenulum accommodation zone 14B for receiving frenulum F.
FIG. 4A shows point F and incisor 15A, with tongue guide 15 having a long taper resulting in a gradual sloped ramp.
FIG. 4B shows point F and incisor 15A, with tongue guide 15 having a shorter taper relative to FIG. 4A, which of course means steeper ramp.
FIG. 4C shows point F and incisor 15A, with tongue guide 15 having a concave ramp.
FIG. 4D shows point F and incisor 15A, with tongue guide 15 having a convex ramp.
FIG. 4E shows point F and incisor 15A, with tongue guide 15 having a convex ramp.
FIG. 4F shows point F and incisor 15A, with tongue guide 15 extending close to point F and having a blunt nosed ramp.
FIG. 4G shows point F and incisor 15A, with tongue guide 15 being short and having a blunt nosed ramp.
FIG. 5A shows point F and incisor 15A, with tongue guide 15 being ½ the height of incisor 15.
FIG. 5B shows point F and incisor 15A, with tongue guide 15 being ¾ the height of incisor 15.
FIG. 5C shows point F and incisor 15A, with tongue guide 15 being the height of incisor 15.
FIG. 5D shows point F and incisor 15A, with tongue guide 15 being higher than the height of incisor 15.

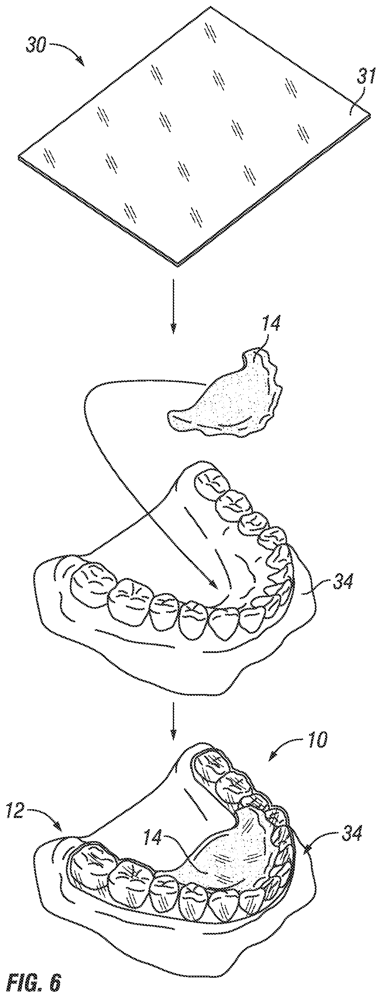

FIG. 6 is a schematic representation of a non-limiting vacuum forming method 30 for manufacturing the tongue guiding apparatus of the present invention.
Referring now to FIG. 7, there is shown a progression of appliances 10A, 10B, 10C and 10D, representing appliances for a succession of treatment steps.
FIG. 8 shows a non-limiting embodiment of appliance 10 that may include a supporting shell member 12 that engages the lower set of teeth 15 in a human mouth, a tongue guide member 14 supported by the shell member, and at least one bite block 35 supported by the supporting shell member 14.
FIG. 9 is a schematic representation of a non-limiting vacuum forming method 30 for manufacturing a bite block embodiment of the tongue guiding apparatus of the present invention, with it understood that this is the same method 30 as shown in FIG. 6, except that tongue guiding member 14 further comprises at least one bite block.
FIG. 10 shows a non-limiting embodiment of appliance 10 that may include a supporting shell member 12 that engages the lower set of teeth 15 in a human mouth, a tongue guide member 14 supported by the shell member, and at least one bite block 35 on each side of supporting shell member 14.
FIG. 11 shows a non-limiting embodiment of appliance 10 that may include a supporting shell member 12 that engages the lower set of teeth 15 in a human mouth, a tongue guide member 14 supported by the shell member, and at least one bite block 35 on each side of supporting shell member 14.
FIG. 12 shows a non-limiting embodiment of appliance 10 that may include a supporting shell member 12 that engages the lower set of teeth 15 in a human mouth, a tongue guide member 14 supported by the shell member, and at least one bite block 35 on each side of supporting shell member 14.
FIG. 13A shows a non-limiting appliance system 100 having for the bottom teeth an appliance 10 that is as described above and that may or may not include a top supporting shell 14T for the top teeth, with at least one bite block 35 is shown positioned on the left side (relative to the patient's mouth) of bottom supporting shell member 14.
FIG. 13B shows a non-limiting appliance system 100 having for the bottom teeth an appliance 10 that is as described above and that may or may not include a top supporting shell 14T, with at least one bite block 35 is shown positioned on the right side of bottom supporting shell member 14.
FIG. 13C shows a non-limiting appliance system 100 having for the bottom teeth an appliance 10 that is as described above and that may or may not include a top supporting shell 14T, with at least two bite blocks 35 are shown positioned on the left side of bottom supporting shell member 14. Certainly, more bite blocks 35 may be positioned on either top supporting shell member 14T and/or bottom supporting shell member 14.
FIG. 13D a non-limiting appliance system 100 having for the bottom teeth an appliance 10 that is as described above and that may or may not include a top supporting shell 14T, with at least an elongated bite block 35 is shown positioned on the left side of bottom supporting shell member 14.
FIG. 13E shows a non-limiting appliance system 100 having for the bottom teeth an appliance 10 that is as described above and that may or may not include a top supporting shell 14T, with at least bite blocks 35 are shown positioned on the left side of supporting member 14 and bite block 35 is shown positioned on the right side of bottom supporting shell member 14.
FIG. 13F shows a non-limiting appliance system 100 having for the bottom teeth an appliance 10 that is as described above and that may or may not include a top supporting shell 14T, with at least a bite block 35 is shown positioned on both left and right side of supporting member 14.
FIG. 13G shows a non-limiting appliance system 100 having for the bottom teeth an appliance 10 that is as described above and having a top supporting shell 14T, with at least a bite block 35 is shown positioned on left side of supporting member 14, and on the left side of top supporting shell 14T.
FIG. 13H shows a non-limiting appliance system 100 having for the bottom teeth an appliance 10 that is as described above and having a top supporting shell 14T, with at least a bite block 35 is shown positioned on left side of supporting member 14, and on the right side of top supporting shell 14T, that is the bite blocks 35 are in a diagonal relationship across the mouth. It should be understood that a diagonal relationship could be established by moving the each block to the opposite side of the mouth.

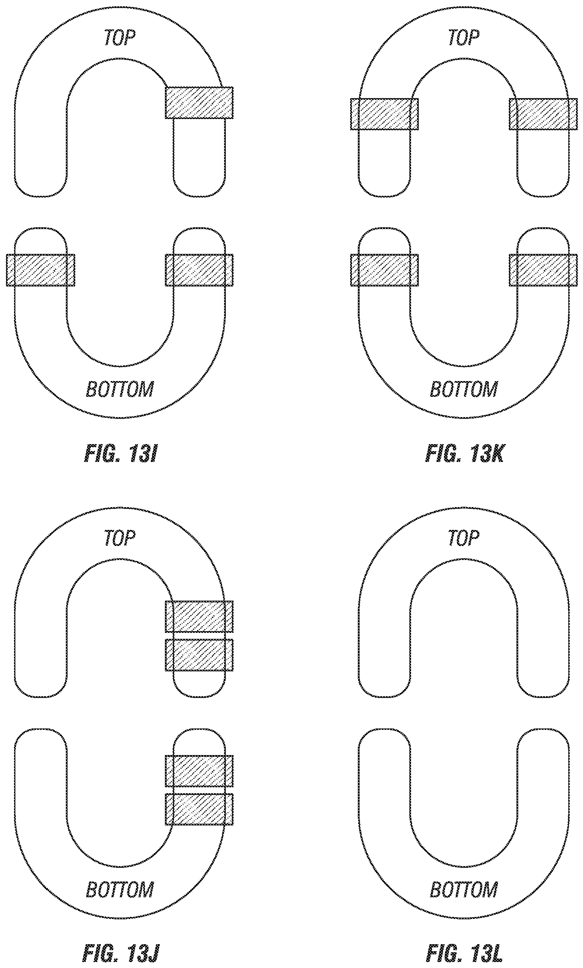

FIG. 13I shows a non-limiting appliance system 100 having for the bottom teeth an appliance 10 that is as described above and having a top supporting shell 14T, with at least a bite block 35 is positioned on both sides of supporting member 14, and on the left side of top supporting shell 14T. It should be understood bite block 35 on the left side of shell 14T could be repositioned on the right side of shell 14T. It should also be understood, that either of the bite blocks on bottom shell 14 could be removed, with a bite block 35 provided on both the left and right side of top shell 14T. The idea is that one side of one of the shells is lacking a bite block.
FIG. 13J shows a non-limiting appliance system 100 having for the bottom teeth an appliance 10 that is as described above and having a top supporting shell 14T, with the idea is that bite blocks 35 are positioned the left or right side of supporting member 14 (left as shown in FIG. 13J), and on the left or right side of top supporting shell 14T (left as shown in FIG. 13J).
FIG. 13K shows a non-limiting appliance system 100 having for the bottom teeth an appliance 10 that is as described above and having a top supporting shell 14T, with at least the idea is that a bite blocks 35 is positioned on both the left and right side of both supporting member 14 and top supporting shell 14T.
FIGs. 14A, 14B and 14C show various bite blocks 35 having one or more tapered sides 39 that upon vacuum formation of a supporting shell member therearound, will urge bite block 35 and the supporting shell member to stay affixed together.
FIG. 14D shows bite block 35 having at least one surface protrusion 36 that that upon vacuum formation of a supporting shell member therearound, will urge bite block 35 and the supporting shell member to stay affixed together.
FIG. 14E shows bite block 35 with a concave surface 37 that that upon vacuum formation of a supporting shell member therearound, will urge bite block 35 and the supporting shell member to stay affixed together.
FIG. 14F shows bite block 35 with a ridged surface 37 that that upon vacuum formation of a supporting shell member therearound, will urge bite block 35 and the supporting shell member to stay affixed together.
FIG. 15 shows a system 210 of the present invention that comprises an orthodontic appliance 201 (comprising at least one of an upper appliance 201U and a lower appliance 201L) for moving teeth that is worn during a time period, and that comprises a lingual positioning appliance 10 (as described above) that is worn during an alternate time period.

### DETAILED DESCRIPTION OF THE INVENTION

It is to be understood that the following disclosure provides many different non-limiting embodiments, or examples, for implementing different features of various non-limiting embodiments. Specific examples of compositions are described below to simplify the disclosure and are not intended to limit the scope of the claims. These are, of course, merely examples and are not intended to be limiting of the scope of the claims. The section headings used herein are for organizational purposes and are not to be construed as limiting the subject matter described.

### Non-Limiting Apparatus of the Present Invention

The present invention provides, among other things, a lingual positioning device. Some non-limiting embodiments of the tongue guiding apparatus of the present invention are for guiding the tongue into a desired position. In even other non-limiting embodiments of the present invention, a tongue guiding apparatus is provided that urges the tongue upward toward the roof of the mouth. In still other non-limiting embodiments of the present invention, multifunction equipment is provided that will not only provide for jaw expansion, but at the same time urge/move the teeth into or toward their desired position, that is, from an initial tooth arrangement to a desired (i.e. final) tooth arrangement.

Some non-limiting embodiments of the tongue guiding apparatus of the present invention may include a supporting shell member that comprises a member that engages the lower set of teeth in a human mouth, and another member that engages and guides the tongue.

It should be understood any of the embodiments shown herein as having a tongue guide and/or bite block, may be provided with or without the tongue guide and/or bite block.

Some non-limiting embodiments of the present invention comprise systems that comprise an orthodontic appliance for moving teeth that is worn during a time period, and that comprise a lingual positioning appliance that is worn during an alternate time period. These systems may comprise a series of orthodontic appliances, and a series of lingual positioning appliances. Most conveniently, orthodontic appliance is an aligner, most likely a clear aligner. Such aligners are well known in the dental arts. As non-limiting examples, any of the aligners as shown in any of the prior art publications and patents cited herein are incorporated by reference. Further, any of the many commercially available clear aligners are believed to be suitable for use as the orthodontic appliance in the present invention. An example of a suitable commercially available aligner useful in the present invention includes CLEARCORRECT^{®} clear aligners, available from the Straumann Group.

The tongue guide device of the present invention is designed to be worn by a patient, causing the tongue to be urged toward the roof of the mouth. Without being limited/bound by theory, the inventor believes that this causes the tongue to press against the lingual side of the incisors on the upper arch. The inventor also believes that this helps with breathing, because the tongue against the top of the mouth causes a seal encouraging/helping the patient to breathe through the nose. The inventor also believes this pressing against those incisors will cause the teeth to move forward, reshaping/moving the upper jaw in the process, subsequently allowing the lower jaw more room to move forward. The inventor also believes that this in turn will reshape/enlarge the airway.

It's probably most convenient for most patients to wear the apparatus of the present invention at night when sleep and breathing disorders are a problem. The present device may also be worn during the day. It is also believed that intermittent wearing will elicit the most movement from the teeth/jaw. For most patients wearing the device at night and allowing daytime for rest will work sufficiently.

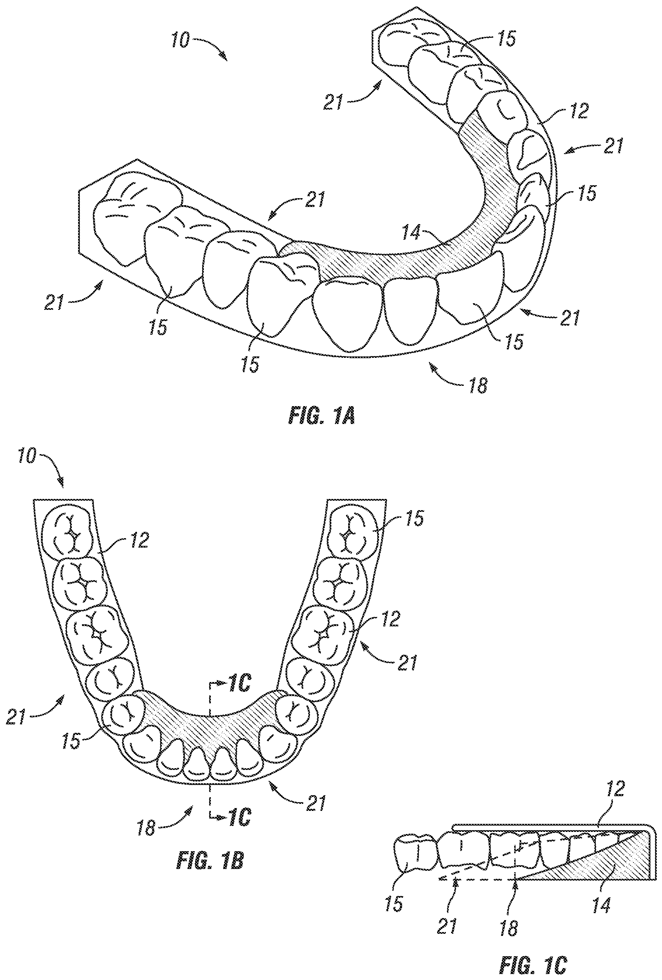

Referring now to FIG. 1A, FIG. 1B, and FIG. 1C, there are shown, respectively, isometric, top, and cross-sectional views of one non-limiting embodiment of tongue guiding apparatus 10 of the present invention positioned on a lower set of teeth 18 comprising one or more teeth 15. This tongue guiding apparatus 10 comprises a supporting shell member 12, and a tongue guide member 14. FIG. 1C is a cutaway view from FIG. 1B as shown.

While guide member 14 is shown in FIGs. 1A, 1B and 1C as being underneath supporting shell potion 12 it should be understood in other embodiments of tongue guiding apparatus 10 that guide member 14 is supported on top of supporting shell member 12. It should also be understood that in other embodiments of tongue guiding apparatus 10 that guide member 14 is incorporated within supporting shell member 12 as a distinct member. It should also be understood that in other embodiments of tongue guiding apparatus 10 that guide member 14 and supporting shell member 12 are fully integrated as one piece.

It should be understood that for some non-limiting embodiments of the present invention, the tongue guiding apparatus of the present invention is designed for a specific patient, in particular, for that patient's lower set of teeth. That is, the apparatus will comprise geometry that is the negative of the geometry of the patient's teeth. It should also be understood, that for other non-limiting embodiments of the present invention, the tongue guiding apparatus of the present invention may be designed to be one size fits many or perhaps all patients. That is, such a device is more of a general device that comprises for generalized geometry to be able to accommodate a number of patients having a wide range of tooth arrangements more along the idea of an athletic mouthpiece.

Supporting shell member 12 may comprise a shell having at least one tooth-receiving cavity 21 formed therein. Supporting shell member 12 is designed to fit over one or more teeth 15 of the lower set of teeth 18. More specifically, supporting shell member 12 is designed to fit over 1, 2, 3, 4, 5, 6, 7, 8, 9, 10, 11, 12, 13, 14, or 15 or more of the teeth 15 of the lower set of teeth 18. Some non-limiting embodiments of supporting shell member 12 are designed to fit over all of a patient's teeth 15 of the lower set of teeth 18 (which number "all" may vary according to any specific patient).

Supporting shell member 12 will also comprise one or more tooth receiving concave cavities 21, having geometries that conform to the teeth that are to be received within those cavities. Specifically, those geometries are negative geometries of the teeth that are to be received therein. Those cavities 21 will generally snuggly receive its corresponding tooth 15, generally forming a friction fit. More specifically, Supporting shell member 12 will also comprise 1, 2, 3, 4, 5, 6, 7, 8, 9, 10, 11, 12, 13, 14, or 15 or more tooth receiving concave cavities 21, having geometries that conform to the teeth that are received within those cavities.

Supporting shell member 12 will generally be made of a suitable polymeric material as are well known in the dental arts. Any suitable thermoplastic or thermoset material may be utilized.

As can be noticed in FIGs. 1-3, tooth guide 14 will have a shape and position that will urge the tongue upwards toward the roof of the mouth. In general, tooth guides of the present invention may be any suitable regular or irregular geometric shape, or suitable any curvilinear shape. The tooth guide must fit under the tongue and not unduly impinge upon the frenulum of the tongue.

The human anatomy includes the frenulum which is a small fold of mucous membrane extending from the floor of the mouth to the midline of the underside of the tongue. It should be understood, that the position and extent of the frenulum varies from patient to patient, and representations of the frenulum in any of the drawings should be understood as a representative position and that the exact position for any given patent may or may not correspond as shown in any of these drawings. It should also be understood that while the frenulum is shown as a point F in some of the drawings, it has a curvilinear shape from the floor of the mouth to the midline of the underside of the tongue.

In most embodiments, it should be understood the design/shape of the tooth guide is determined by the shape of the lingual side of the teeth and the position of the frenulum.

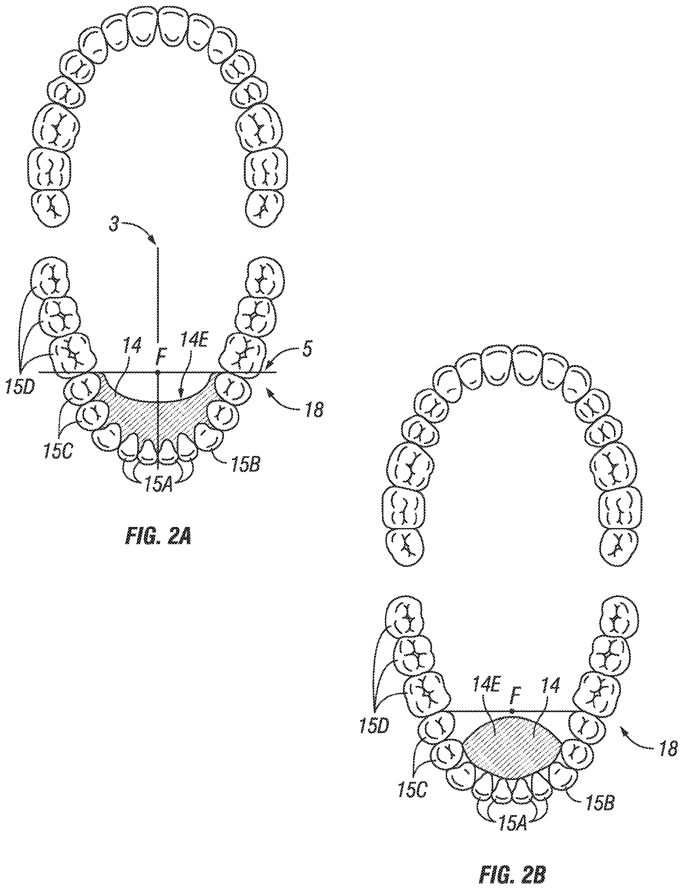

As a non-limiting example, in some non-limiting embodiments, the tooth guide will extend away from the lingual side of the teeth toward the frenulum but not past the frenulum. See, FIG. 2A, showing a lower set of teeth 18 with incisors 15A, canines 15B, premolars 15C, and molars 15D, point F where and point F where tongue guide 14 will impinge on the frenulum. In most patient anatomies, the frenulum will reside along a centerline 3. Line 5 is perpendicular to centerline 3 and through point F. This centerline 3 and line 5 are either shown or should be assumed on all other drawings.

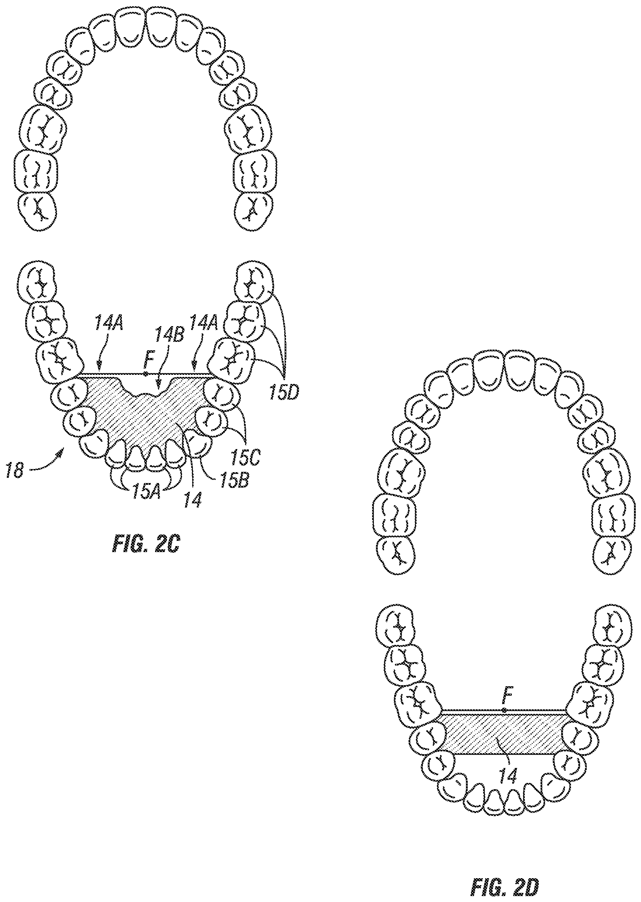

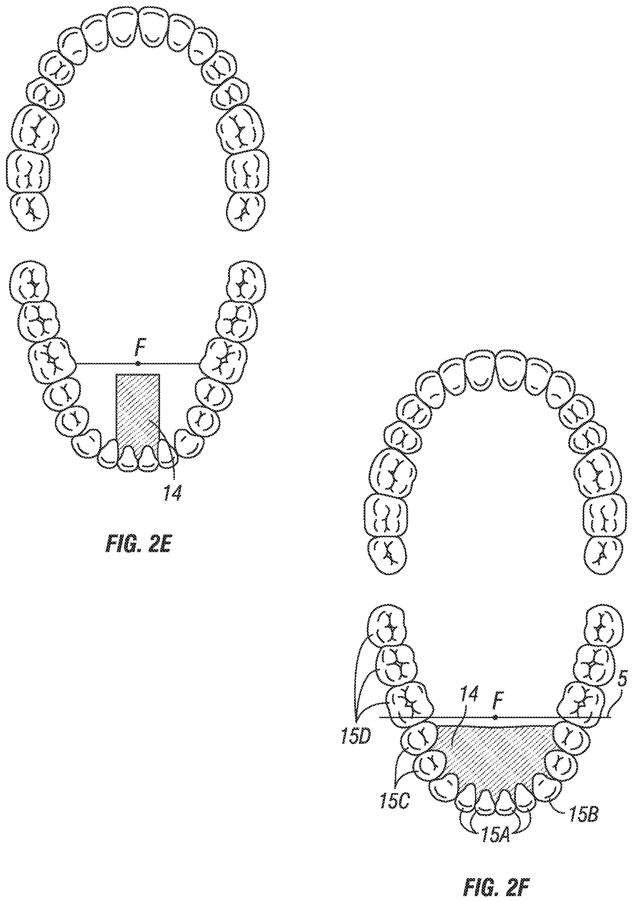

As another non-limiting example, in some non-limiting embodiments, the tooth guide will extend away from the lingual side of the teeth toward the frenulum but not past the frenulum. As non-limiting examples, see, FIGs. 2A, 2B, 2C, 2D and 2E, all showing a lower set of teeth 18 with incisors 15A, canines 15B, premolars 15C, and molars 15D, point F where and point F where tongue guide 14 will impinge on the frenulum.

Specifically, referring now to FIG. 2A, tongue guide 14 extends from the front teeth (i.e., incisors 15A and others) back toward but not impinging upon frenulum, with an edge 14E facing the frenulum that is concave relative to the frenulum.

Specifically, referring now to FIG. 2B, tongue guide 14 extends from extends from the front teeth (i.e., incisors 15A and others) back toward and impinging upon frenulum F, with an edge 14E facing the frenulum that is convex relative to the frenulum.

Specifically, referring now to FIG. 2C, tongue guide 14 extends from extends from the front teeth (i.e., incisors 15A and others) back toward frenulum F, with a frenulum receiving zone 14B defined between tongue guide extensions 14A (which do not extend past frenulum F), with the edge facing the frenulum having both concave and convex portions relative to the frenulum.

Specifically, referring now to FIG. 2D, tongue guide 14 extends across the premolars and/or molars, not in contact with the front incisors 15A, and not impinging upon frenulum F.

Specifically, referring now to FIG. 2E, tongue guide 14 extends from the front incisors 15A and back toward but not impinging upon frenulum F.

Specifically, referring now to FIG. 2F, tongue guide 14 extends from extends from the front teeth (i.e., incisors 15A and others) back toward frenulum F, with a frenulum receiving zone 14B defined between tongue guide extensions 14A (which do not extend past frenulum F), with the edge facing the frenulum presenting a flat face relative to the frenulum.

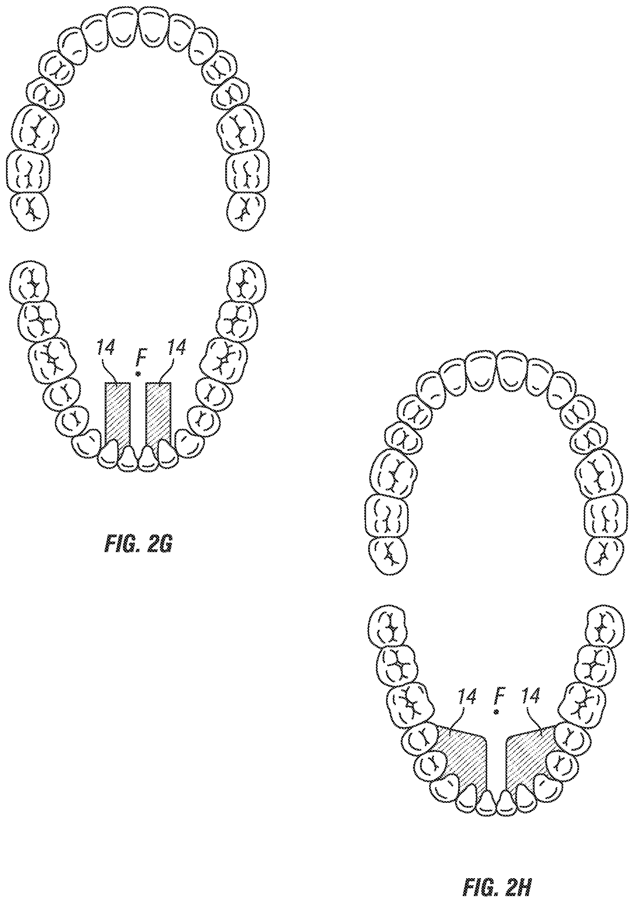

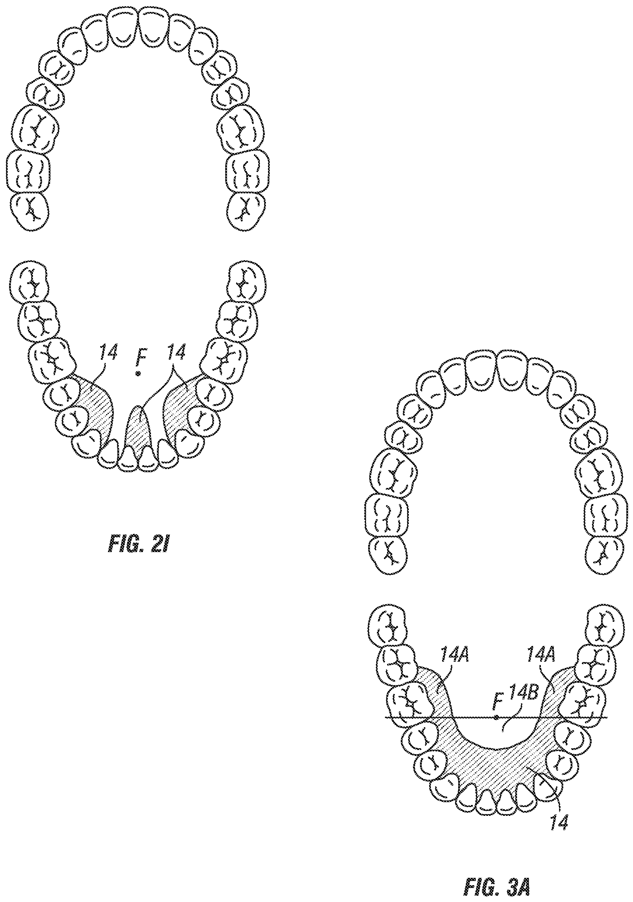

Specifically, referring now to FIGs. 2G, 2H, and 2I, multiple tongue guides 14 extend from the lingual side of the teeth back toward but not impinging upon frenulum F (which do not extend past frenulum F). It should be understood any suitable number of tongue guides may be employed for use in the present invention.

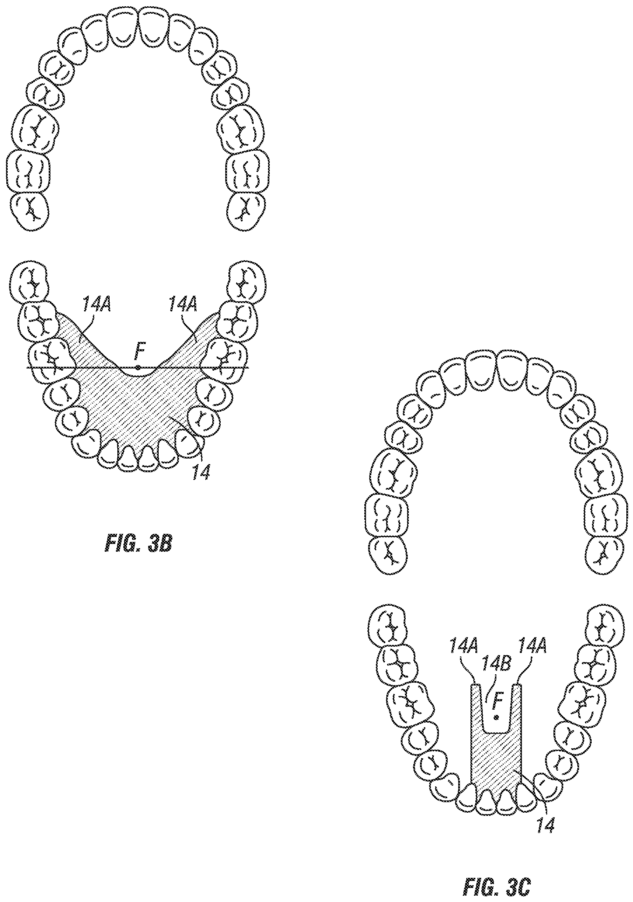

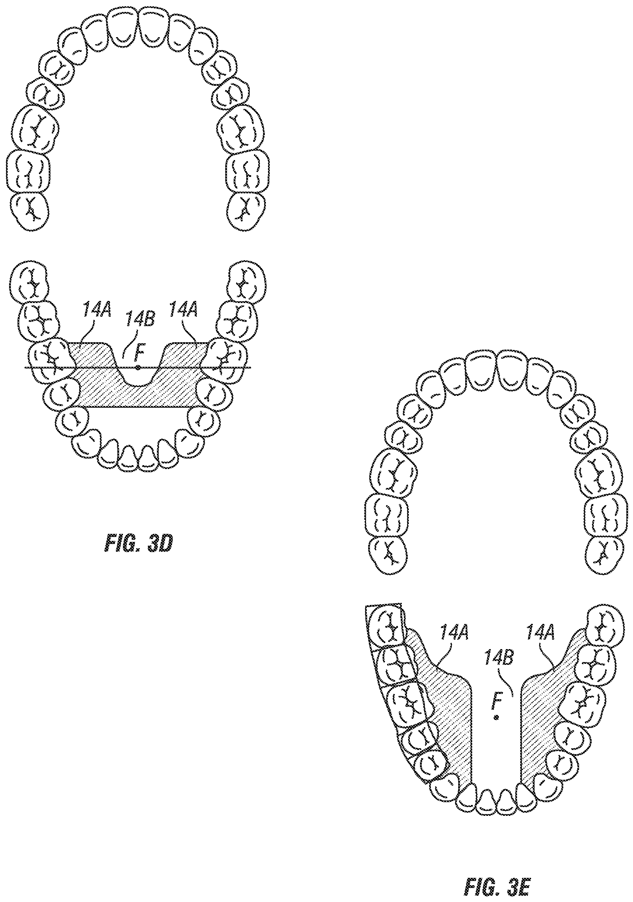

As another non-limiting example, in some non-limiting embodiments, the tooth guide will extend away from the lingual side of the teeth toward the frenulum, with some of the tooth guide extending past but not impinging upon the frenulum. As non-limiting examples, see, FIGs. 3A, 3B, 3C, 3D and 3E, all showing a lower set of teeth 18 with incisors 15A, canines 15B, premolars 15C, and molars 15D, point F where and point F where tongue guide 14 will impinge on the frenulum.

Specifically, referring now to FIG. 3A, tongue guide 14 comprises extensions 14A which extend past each side of frenulum F to define a frenulum accommodation zone 14B for receiving frenulum F.

Specifically, referring now to FIG. 3B, tongue guide 14 comprises extensions 14A which extend past each side of frenulum F to define a frenulum accommodation zone 14B for receiving frenulum F, with impingement upon frenulum F.

Specifically, referring now to FIG. 3C, tongue guide 14 only extends from the front teeth (incisors 15A), and comprises extensions 14A which extend past each side of frenulum F to define a frenulum accommodation zone 14B for receiving frenulum F.

Specifically, referring now to FIG. 3D, tongue guide 14 extends across the lower teeth 18, and comprises extensions 14A which extend past each side of frenulum F to define a frenulum accommodation zone 14B for receiving frenulum F.

Specifically, referring now to FIG. 3E, tongue guide 14 comprises extensions 14A which extend past each side of frenulum F to define a frenulum accommodation zone 14B for receiving frenulum F.

It should be understood that any of the shapes shown in FIGs. 2A-I and 3A-E can be utilized for tongue guide 14 whether or not the tongue guide 14 extends past the frenulum or not.

It should also be understood that the shape of tongue guide 14 is not to be limited to any of those as show in FIGs. 2A-I and 3A-E.

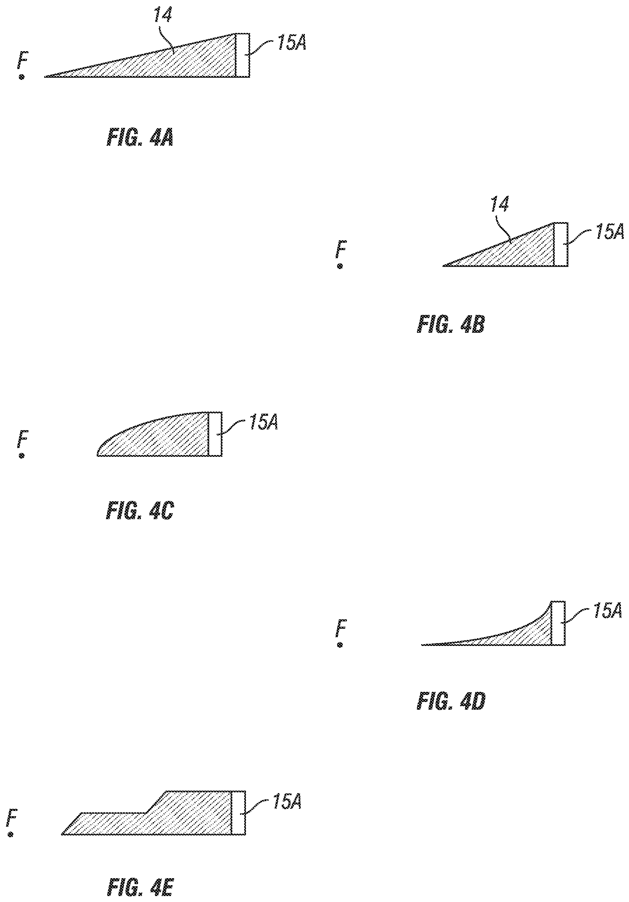

Referring again to FIG. 1C, which is a cutaway view from FIG. 1B as shown, it should be understood that tongue guide 14 may employ any suitable cross-sectional shape. Referring now to FIGs 4A-G, there are shown various non-limiting embodiments of suitable cross-sectional shapes for tongue guide 14.

Specifically, FIG. 4A shows point F and incisor 15A, with tongue guide 15 having a long taper resulting in a gradual sloped ramp.

Specifically, FIG. 4B shows point F and incisor 15A, with tongue guide 15 having a shorter taper relative to FIG. 4A, which of course means steeper ramp.

Specifically, FIG. 4C shows point F and incisor 15A, with tongue guide 15 having a concave ramp.

Specifically, FIG. 4D shows point F and incisor 15A, with tongue guide 15 having a convex ramp.

Specifically, FIG. 4E shows point F and incisor 15A, with tongue guide 15 having a convex ramp.

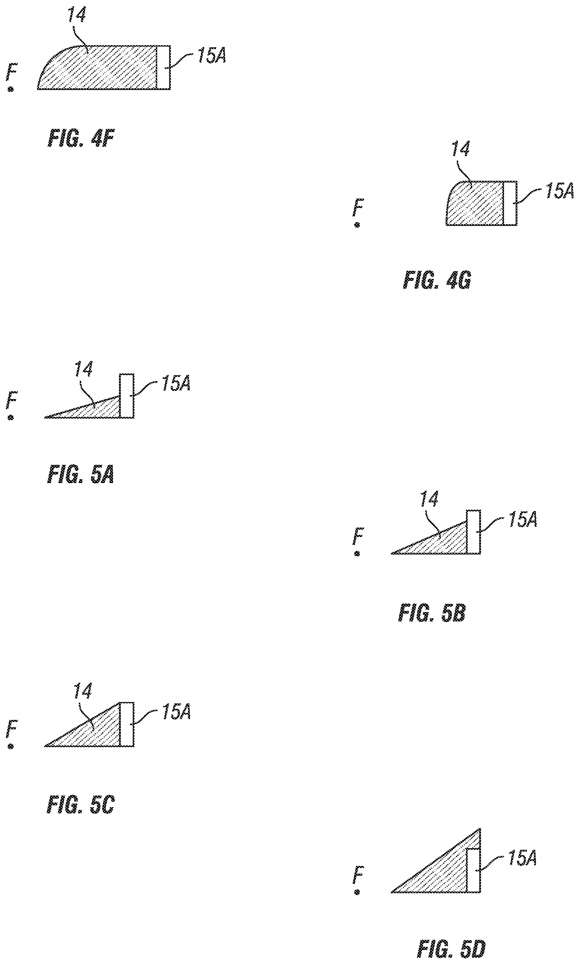

Specifically, FIG. 4F shows point F and incisor 15A, with tongue guide 15 extending close to point F and having a blunt nosed ramp.

Specifically, FIG. 4G shows point F and incisor 15A, with tongue guide 15 being short and having a blunt nosed ramp.

It should also be understood that tongue guide 15 will raise the tongue to various heights relative to the incisors, for example, ¼, ½, ¾ the height of the incisors, as well as incisor height, or even over the incisor.

See, FIGs. 5A-5C, showing various heights of tongue guide 15.

Specifically, FIG. 5A shows point F and incisor 15A, with tongue guide 15 being ½ the height of incisor 15.

Specifically, FIG. 5B shows point F and incisor 15A, with tongue guide 15 being ¾ the height of incisor 15.

Specifically, FIG. 5C shows point F and incisor 15A, with tongue guide 15 being the height of incisor 15.

Specifically, FIG. 5D shows point F and incisor 15A, with tongue guide 15 being higher than the height of incisor 15.

In some non-limiting embodiments, tongue guiding apparatus 10 is designed not only to position the tongue but is further designed to also reposition at least one of the patient's teeth. Specifically, supporting shell member 12 may also comprise at least one concave cavity 21 that is designed to reposition a tooth. Thus, supporting shell member 12 may comprise tooth receiving geometry to reposition the teeth to a different tooth arrangement.

In such a tooth repositioning embodiment, the tongue guiding appliance 10 will be configured to have at least one tooth-receiving cavity has a geometry causing a tooth to move to a new position relative to its current position. Is should be understood that in treatment, not all teeth are moved at each step of treatment. Generally, when such an appliance is first worn by the patient, one or more of the teeth will be misaligned relative to an undeformed geometry of the appliance cavity. The appliance, however, is sufficiently resilient to accommodate or conform to the misaligned teeth, and will apply sufficient resilient force against such misaligned teeth in order to reposition the teeth to the new position for that treatment step (be it an intermediate or final positioning).

Thus, such tooth moving tongue guiding systems according to the present invention may include one or more such tongue guiding appliances 10 having a geometry selected to reposition a patient's teeth to a new position for that treatment step. Such systems may include 1, 2, 3, 4, 5, 6, 7, 8, 9, 10, 11, 12, 13, 14, 15 or more tongue guiding appliances 10 to incrementally reposition teeth. Thus, it should be understood such systems of tongue guiding appliances 10 may incrementally reposition teeth from an initial tooth arrangement to a desired/final tooth arrangement.

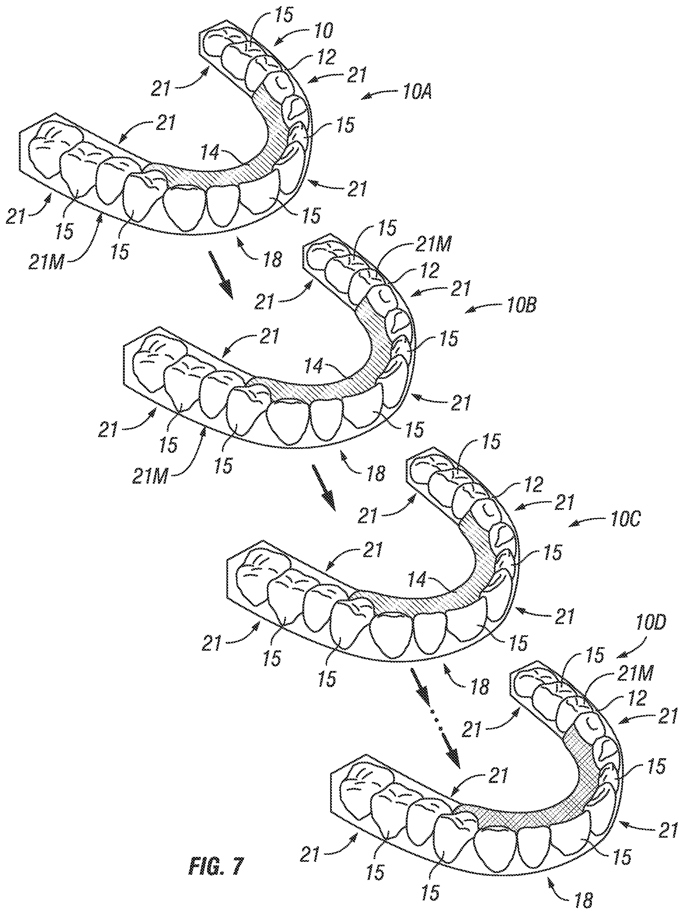

Referring now to FIG. 7, there is shown a progression of appliances 10A, 10B, 10C and 10D, representing appliances for a succession of treatment steps. Again, it should be understood that any system of the present invention may comprise 1 or more appliances and is not limited to any particular number. It should be understood, that these appliances 10A, 10B, 10C and 10D are the same as appliance 10 described above, except for the presence in appliances 10A, 10B and 10D of tooth cavity 21M having a geometry that will urge/move a tooth into a new position for the treatment step, and in 10D the absence of tongue positioning member 14. Specifically, apparatus 10A and 10B, are all multipurpose tooth repositioning and tongue guiding apparatus, comprising a tongue guide member 14, and a supporting shell member 12 having at least one tooth cavity 21 having a geometry that will urge/move a tooth into a new position for the treatment step. Apparatus 10C is intentionally only positioning the tongue, and not moving any teeth. In this embodiment as shown, there may or may not be more apparatus/steps between apparatus C and apparatus D. Apparatus D is intentionally only repositioning teeth and not positioning the tongue. Thus, it should be understood some treatment systems may or may not include tooth positioning at each treatment step. It should also be understood that some treatment systems may or may not include tooth movement. It should also be understood that treatment systems of the present invention will involve tongue positioning in at least one treatment step, and/or tooth repositioning in at least one treatment step.

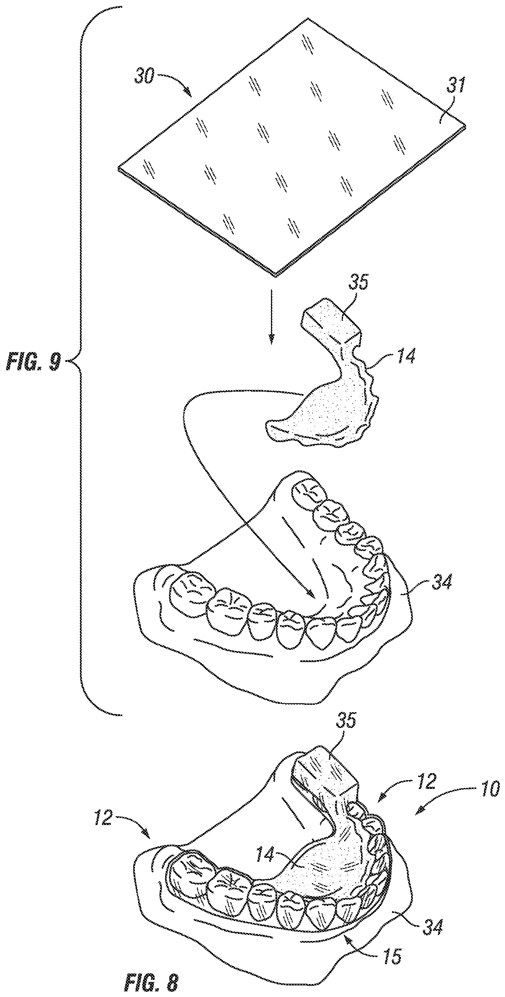

Some non-limiting embodiments of the apparatus of the present invention may include at least one bite block, and may or may not also include a tongue guide member Referring now to FIG. 8, there is shown a non-limiting embodiment of one such appliance 10 that may include a supporting shell member 12 that engages the lower set of teeth 15 in a human mouth, a tongue guide member 14 supported by the shell member, and at least one bite block 35 supported by the supporting shell member 14. While bite block 35 is shown integrated into and part of tongue member 14, in some embodiments, bite block 35 may exist independent of tongue guide member 14.

The various apparatus of the present invention as disclosed herein may also include bite blocks 35 on each side of the apparatus. In fact, observing that an appliance my consist of both upper and lower arch members, any combination of one, two, three, four, or more bite blocks on the left/right side of the upper/lower arch members is contemplated. As non-limiting examples, a bite block on the upper right only, upper left only, lower right only, lower left only, or a bite block on only one of the upper right, upper left, lower right or lower left, in combination with a bite block on one, two or three of the following of lower right, lower left, and upper left. Any of these combinations may or may not be paired with a tongue guide.

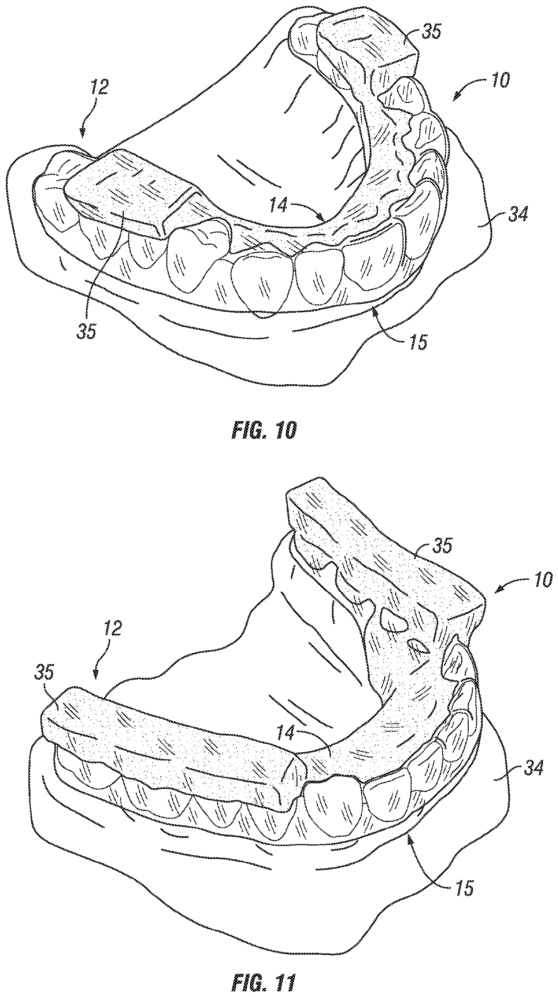

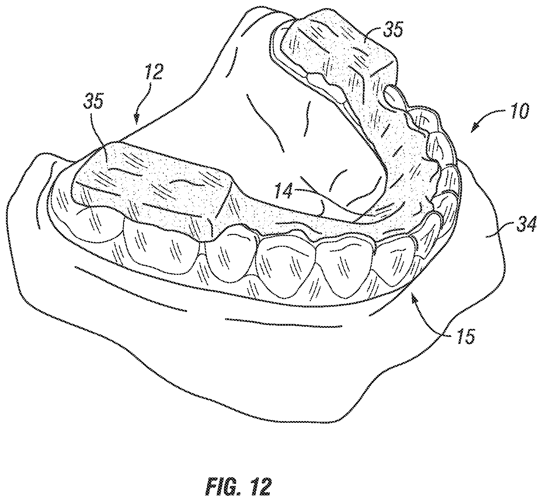

Referring now to FIGs. 10, 11 and 12, there are shown non-limiting embodiments of appliance 10 that may include a supporting shell member 12 that engages the lower set of teeth 15 in a human mouth, a tongue guide member 14 supported by the shell member, and at least one bite block 35 on each side of supporting shell member 14. The lengths of the various bite block 35 are shown as increasing from FIG. 10, to FIG. 12, and then to FIG. 11.

It should be understood that the bite blocks utilized herein may be any suitable shape that is desirable and/or practical, include any regular geometric shape, any n-sided geometric side where n may be 3, 4, 5, 6, 7, 8, 9, 10, 11, 12, or more, any irregular geometric shape, any shape having linear sides, any shape having curvilinear sides, any shape having convex sides, any shape having convex sides, and combinations of the foregoing.

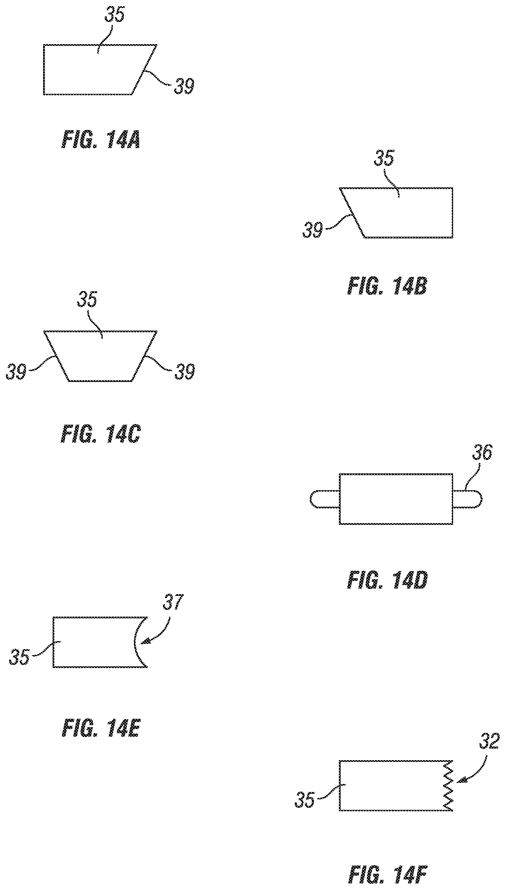

Bite blocks utilized in the present invention will also comprise side walls that urge the bite block to stay affixed to a supporting shell member that has been vacuum formed around the bite block. Referring now to FIGs 14A-14F, there are provides various non-limiting embodiments of cross-sectional views of bite blocks.

Referring now to FIGs.14A-C, there are shown bite blocks 35 having one or more tapered sides 39 that upon vacuum formation of a supporting shell member therearound, will urge bite block 35 and the supporting shell member to stay affixed together.

Referring now to FIG. 14D, there is shown bite block 35, having at least one surface protrusion 36 that that upon vacuum formation of a supporting shell member therearound, will urge bite block 35 and the supporting shell member to stay affixed together. It should be understood that any suitable shape and size of protrusion 36 may be utilized.

Referring now to FIG. 14E, there is shown bite block 35 with a concave surface 37 that that upon vacuum formation of a supporting shell member therearound, will urge bite block 35 and the supporting shell member to stay affixed together.

Referring now to FIG. 14F, there is shown bite block 35 with a ridged surface 37 that that upon vacuum formation of a supporting shell member therearound, will urge bite block 35 and the supporting shell member to stay affixed together.

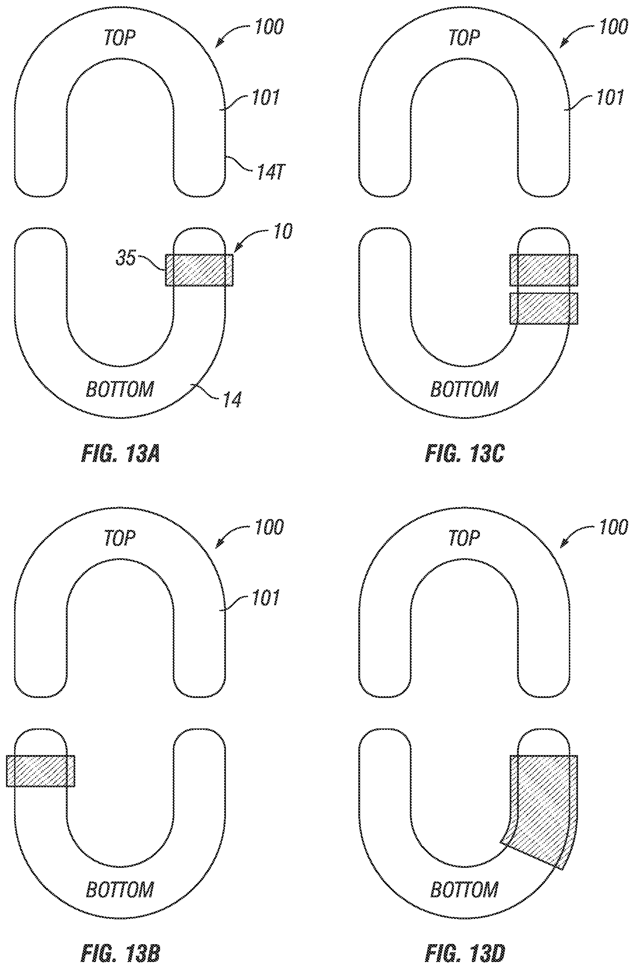

FIGs 13A to 13K show various non-limiting embodiments of bite block configurations of various appliance systems 100. In each appliance system 100 there is a bottom appliance 10 for the patient's bottom teeth that is as described above with its supporting shell. Each appliance system 100 may or may not further include a top supporting shell 14T for the patient's top teeth. It should be understood appliance system 100 may include one or more bite blocks 35 positioned anywhere on the biting surface of top supporting shell 14T and/or the biting surface of bottom supporting shell 14. The top shell 14T comprises at least one tooth receiving geometry for receiving at least one of the teeth in the top jaw of a patient's mouth.

Appliance suite 100 may include only one bite block on bottom supporting shell 14, only one bite block on top supporting shell 14T, bite blocks on bottom supporting shell 14, more bite blocks on top supporting shell 14T, bite blocks on both shell 14 and shell 14T, bite blocks on both sides of bottom supporting shell 14, bite blocks on both sides of bottom supporting shell 14T, at least one bite block on both bottom supporting shell 14 and top supporting shell 14T, only one bite block on bottom supporting shell 14 and bite blocks on top supporting shell 14T, bite blocks on bottom supporting shell 14 and only one bite block on top supporting shell 14T, or bites block on both bottom supporting shell 14 and top supporting shell 14T.

Referring now to FIG. 13A, there is shown a non-limiting appliance system 100 having for the bottom teeth an appliance 10 that is as described above and that may or may not include a top supporting shell 14T for the top teeth. At least one bite block 35 is shown positioned on the left side (relative to the patient's mouth) of bottom supporting shell member 14. Certainly, more bite blocks 35 may be positioned on either top supporting shell member 14T or bottom supporting shell member 14.

Referring now to FIG. 13B, there is shown a non-limiting appliance system 100 having for the bottom teeth an appliance 10 that is as described above and that may or may not include a top supporting shell 14T. At least one bite block 35 is shown positioned on the right side of bottom supporting shell member 14. Certainly, more bite blocks 35 may be positioned on either top supporting shell member 14T and/or bottom supporting shell member 14.

Referring now to FIG. 13C, there is shown a non-limiting appliance system 100 having for the bottom teeth an appliance 10 that is as described above and that may or may not include a top supporting shell 14T. At least two bite blocks 35 are shown positioned on the left side of bottom supporting shell member 14. Certainly, more bite blocks 35 may be positioned on either top supporting shell member 14T and/or bottom supporting shell member 14.

Referring now to FIG. 13D, there is shown a non-limiting appliance system 100 having for the bottom teeth an appliance 10 that is as described above and that may or may not include a top supporting shell 14T. An elongated bite block 35 is shown positioned on the left side of bottom supporting shell member 14. Certainly, more bite blocks 35 of any size/shape may be positioned on either top supporting shell member 14T and/or bottom supporting shell member 14.

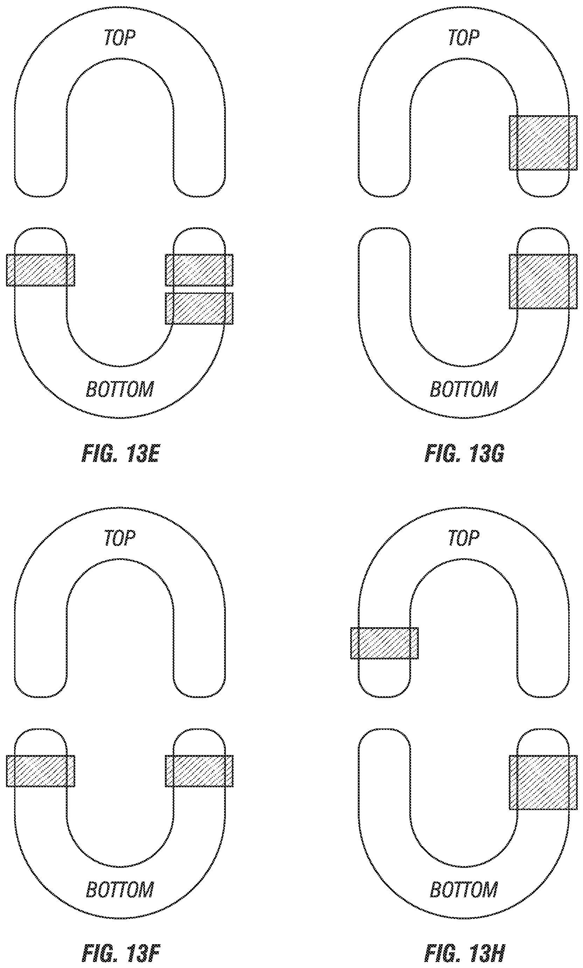

Referring now to FIG. 13E, there is shown a non-limiting appliance system 100 having for the bottom teeth an appliance 10 that is as described above and that may or may not include a top supporting shell 14T. Bite blocks 35 are shown positioned on the left side of supporting member 14 and bite block 35 is shown positioned on the right side of bottom supporting shell member 14. Certainly, more bite blocks 35 of any size/shape may be positioned on either top supporting shell member 14T and/or bottom supporting shell member 14.

Referring now to FIG. 13F, there is shown a non-limiting appliance system 100 having for the bottom teeth an appliance 10 that is as described above and that may or may not include a top supporting shell 14T. A bite block 35 is shown positioned on both left and right side of supporting member 14. Certainly, more bite blocks 35 of any size/shape may be positioned on either top supporting shell member 14T and/or bottom supporting shell member 14.

Referring now to FIG. 13G, there is shown a non-limiting appliance system 100 having for the bottom teeth an appliance 10 that is as described above and having a top supporting shell 14T. A bite block 35 is shown positioned on left side of supporting member 14, and on the left side of top supporting shell 14T. Certainly, more bite blocks 35 of any size/shape may be positioned on either top supporting shell member 14T and/or bottom supporting shell member 14.

Referring now to FIG. 13H, there is shown a non-limiting appliance system 100 having for the bottom teeth an appliance 10 that is as described above and having a top supporting shell 14T. A bite block 35 is shown positioned on left side of supporting member 14, and on the right side of top supporting shell 14T, that is the bite blocks 35 are in a diagonal relationship across the mouth. It should be understood a diagonal relationship could be established by moving each block to the opposite side of the mouth. Certainly, more bite blocks 35 of any size/shape may be positioned on either top supporting shell member 14T and/or bottom supporting shell member 14.

Referring now to FIG. 13I, there is shown a non-limiting appliance system 100 having for the bottom teeth an appliance 10 that is as described above and having a top supporting shell 14T. A bite block 35 is positioned on both sides of supporting member 14, and on the left side of top supporting shell 14T. It should be understood bite block 35 on the left side of shell 14T could be repositioned on the right side of shell 14T. It should also be understood, that either of the bite blocks on bottom shell 14 could be removed, with a bite block 35 provided on both the left and right side of top shell 14T. The idea is that one side of one of the shells is lacking a bite block. Certainly, more bite blocks 35 of any size/shape may be positioned on either top supporting shell member 14T and/or bottom supporting shell member 14.

Referring now to FIG. 13J, there is shown a non-limiting appliance system 100 having for the bottom teeth an appliance 10 that is as described above and having a top supporting shell 14T. The idea is that bite blocks 35 are positioned the left or right side of supporting member 14 (left as shown in FIG. 13J), and on the left or right side of top supporting shell 14T (left as shown in FIG. 13J). Certainly, more bite blocks 35 of any size/shape may be positioned on either top supporting shell member 14T and/or bottom supporting shell member 14.
Referring now to FIG. 13K, there is shown a non-limiting appliance system 100 having for the bottom teeth an appliance 10 that is as described above and having a top supporting shell 14T. The idea is that a bite blocks 35 is positioned on both the left and right side of both supporting member 14 and top supporting shell 14T. Certainly, more bite blocks 35 of any size/shape may be positioned on either top supporting shell member 14T and/or bottom supporting shell member 14.

In some non-limiting embodiments of the present invention, systems are provided in which the tongue positioning device is paired with an orthodontic device. In particular, the tongue positioning device and the orthodontic device are each worn during different/alternate time periods. As a non-limiting example, the tongue positioning device may be worn during nighttime/sleeping, and the orthodontic device is worn during the day. As another non-limiting example, both the tongue positioning device and the orthodontic device may be worn at different times of the day. Given that the tongue positioning device is useful for sleep/breathing disorders, it is most likely that the tongue positioning device is worn during sleep, however, it is possible that the orthodontic device could also be worn at night too, either exclusively, or both could be worn alternatively.

In some embodiments where the tongue positioning device is paired with an orthodontic device, the tongue positioning device will have the same tooth receiving geometry as the bottom orthodontic device, that is, both the tongue positioning device and the bottom orthodontic device will urge/guide/position the bottom teeth to a certain position/configuration. Thus, the bottom orthodontic device will urge/guide/position the bottom teeth to a certain position during a given time period, and when the tongue positioning device is subsequently worn during a different time period it will not only position the tongue it will also urge/guide/position the bottom teeth to that same certain position/configuration.

In some non-limiting embodiments of the present invention, there is provided a system comprising a series of incremental systems each comprising a tongue positioning device paired with an orthodontic device. This series of incremental systems of paired devices is designed to incrementally reposition teeth from an initial tooth arrangement to a final tooth arrangement. Such systems may comprise a series of 2, 3, 4, 5, 6, 7, 8, 9, 10, 11, 12, 13, 14, 15 or more incremental systems of paired devices.

In these systems for repositioning teeth from an initial tooth arrangement to a final tooth arrangement, repositioning is accomplished with a series of paired appliances configured to receive the teeth in a cavity and incrementally reposition individual teeth in a series of successive steps. The successive use of a number of such appliances permits each appliance to be configured to move individual teeth in small increments, typically less than 2 mm, preferably less than 1 mm, and more preferably less than 0.5 mm. These values refer to the maximum linear translation of any point on a tooth as a result of using a single appliance. The movements provided by successive appliances, of course, will usually not be the same for any specific tooth. Thus, one point on a tooth may be moved by a different distance as a result of the use of one appliance and thereafter moved by a different distance and/or in a different direction by a later appliance.

The individual orthodontic appliances and tongue positioning device preferably comprise a polymeric shell having the teeth-receiving cavity formed therein. Each individual appliance is configured so that its tooth-receiving cavity has a geometry corresponding to the next desired tooth arrangement intended for that appliance. That is, when an appliance is first worn by the patient, certain of the teeth will be misaligned relative to an undeformed geometry of the appliance cavity. The appliance, however, is sufficiently resilient to accommodate or conform to the misaligned teeth and will apply sufficient resilient force against such misaligned teeth in order to reposition the teeth to next tooth arrangement desired for that treatment step.

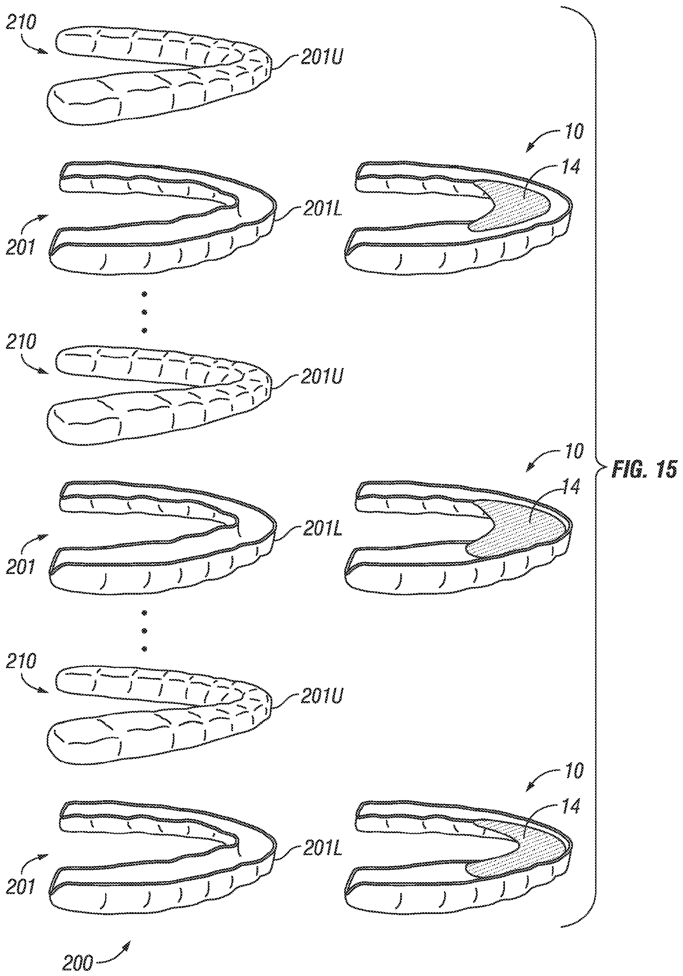

Referring now to FIG. 15, there is shown a system 210 of the present invention that comprises an orthodontic appliance 201 (comprising at least one of an upper appliance 201U and a lower appliance 201L) for moving teeth that is worn during a time period, and that comprises a lingual positioning appliance 10 (as described above) that is worn during an alternate time period. While tongue positioning appliance 10 is shown as a bottom member only, it may be paired with an upper member 14T as described above.

Two or more of these systems 210 may form a system 200 to incrementally move teeth. This system 200 may comprise a series of systems 210. In such a system 200, there are a series of orthodontic appliances 201, and a series of tongue positioning appliances. Most conveniently, orthodontic appliance is an aligner, most likely a clear aligner. Such aligners are well known in the dental arts. As non-limiting examples, any of the aligners as shown in any of the prior art publications and patents cited herein are incorporated by reference. Further, any of the many commercially available clear aligners are believed to be suitable for use as the orthodontic appliance in the present invention. An example of a suitable commercially available aligner useful in the present invention includes CLEARCORRECT^{®} clear aligners, available from the Straumann Group.

### Non-Limiting Methods Of Making the Present Invention

Any of the apparatus as described herein, including the orthodontic device and tongue guide, may be manufactured by any suitable method utilizing any suitable machinery and/or tools as are well known in the dental arts.

As a non-limiting example, part or all of the orthodontic device and/or tongue guiding apparatus may be manufactured completely utilizing techniques that are well known in the dental and manufacturing arts, including but not limited to manual techniques, vacuum forming, molding methods, subtractive methods (milling as a non-limiting example), additive methods (3D printing as a non-limiting example), or combinations thereof.

In various methods of making part or all of the tongue guide apparatus of the present invention, the tongue guide member and the supporting shell member may be made by same or different methods, and at the same or different times.

As a non-limiting example, tongue guide member and supporting shell member can be made unassembled, with the two then assembled together using couplings, adhesives, friction fit, interlocking members, mating members, and the like.

As another non-limiting example, tongue guide member can be made first, with supporting shell member then formed around and affixed to the tongue guide member. As a non-limiting example, vacuum forming or molding the supporting shell member onto/around the tongue guide member. The tongue guide member may have a geometric configuration, surface characteristic, shape, undercut, protrusion, and/or indention, that will allow for supporting shell member to be affixed to the tongue guide member. For example, when the supporting member is vacuum formed around the tongue guide member, the polymer utilized to form the supporting shell member may or may not sufficiently adhere to the tongue guide member. Thus, a mechanical connection must be utilized to affix the two together. Alternatively, part of the supporting shell member may wrap around part of the tongue guide member. Alternatively, the tongue guide member may have a geometric configuration, surface characteristic, shape, undercut, protrusion, that will allow for supporting shell member to be affixed to the tongue guide member. Alternatively, an adhesive may be utilized between the tongue guide member and the supporting shell member.

As even another non-limiting embodiment, the tongue guide member and supporting shell member can be formed together, as non-limiting examples, by additive techniques (as a non-limiting example, 3-D printing the device having both the shell member and the guide member), subtractive techniques (as a non-limiting example, milling the device), and/or molding the device. Any of these techniques may be utilized for making the orthodontic devices of the present invention.

Both the tongue guide member and the supportive shell member, as well as the orthodontic devices, are designed based on a physical and/or digital model (negative or positive) of the patient's teeth. Thus, it is generally desired to obtain digital and/or physical models of the patient's mouth. Various techniques include taking a mold, picture, X-ray, or scan of a patient's teeth, and from that, making a physical and/or digital model of the model of the patient's teeth. Of course, the mold is a negative tooth model from which a positive tooth model may be obtained.

For embodiments in which teeth are to be repositioned in a series of incremental steps form initial tooth positions to final tooth positions, a series of treatment models providing for movement from initial tooth positions to final tooth positions, either physical or most conveniently digital, are obtained from which the systems are made.

The tooth guiding member and/or orthodontic device can then be made from the obtained model of the patient's teeth. It should be understood that the tongue guide member and/or orthodontic device can be digitally designed fully within an information handling system (for example, a computer), or physically designed directly upon a physical model. Methods for obtaining the tongue guide member and/or orthodontic device include designing a digital version of the tooth guiding member (or orthodontic device) and then creating a physical tooth guiding member (or orthodontic device) therefrom, and can also include creating a physical tooth guiding member and/or orthodontic device using a physical model of the teeth.

Bite blocks 35 are shown as being positioned underneath supporting shell member 14. However, regarding bite block embodiment of the tongue guiding apparatus of the present invention, it should be understood, that one or more of the various bite blocks 35 may actually be positioned on top of supporting shell member 14. Rather than the bite block and the supporting shell member being vacuum formed together, the two can be affixed together using methods well known, including using an adhesive, connectors, interlocking members, mating members, friction fit, mechanical fit, and the like,

As another non-limiting example, it is believed that part or all of the tongue guiding apparatus of the present invention may be constructed utilizing rapid prototyping technology. In general, rapid prototyping is a group of techniques used to quickly fabricate a scale model of a physical part or assembly using three-dimensional computer aided design (CAD) data. Construction of part or all of the tongue guiding apparatus of the present invention may be manufactured utilizing one or more additive technologies and/or one or more substractive technologies, generally computer implemented. Such methods includes, but are not limited to 3D printing (3DP), Ballistic particle manufacturing (BPM), Directed light fabrication (DLF), Directshell production casting (DSPC), Fused deposition modeling (FDM), Laminated object manufacturing (LOM), Laminated resin printing (LRP), Shape deposition manufacturing (SDM) (and Mold SDM), Solid ground curing (SGC), Selective laser sintering (SLS), Selective laser melting (SLM), Stereo lithography (SLA), Multi Jet Fusion (MJF), and CNC subtractive technology.

## Claims

1. An oral appliance (10) comprising:
a supporting shell member (12) having concave geometry for receiving at least a portion of a dental arch of a wearer into the concave geometry; and
a tongue guide member (14) extending from said supporting shell member (12) and into contact with an underside of a tongue of said wearer when the portion of the dental arch is received into the concave geometry, wherein the portion of said arch has an arch geometry, and wherein the concave geometry fits over the arch and comprises a negative of at least a portion of the arch geometry, and **characterised in that** the
supporting shell member (12) is configured to fit over the tongue guide member (14).

2. The appliance (10) of claim 1, wherein the arch comprises teeth, with each of the teeth having a unique tooth geometry, and wherein the concave geometry comprises a negative geometry corresponding to the unique tooth geometry of at least one of said teeth.

3. The appliance (10) of claim 2, wherein the tongue guide member (14) comprises a shape that will urge, and is positioned to urge, the tongue upwards toward a roof of a mouth of the wearer.

4. The appliance (10) of claim 3, wherein the teeth have a lingual side, and the tongue guide member (14) will extend away from the lingual side of the teeth.

5. The appliance (10) of claim 4, wherein the teeth comprise one or more incisors, and wherein the tongue guide (14) extends away from the lingual side of incisors.

6. The appliance (10) of claim 4, wherein the teeth comprise one or more incisors and one or more canines, and wherein the tongue guide (14) extends away from the lingual side of the incisors and canines.

7. The appliance (10) of claim 4, wherein the teeth comprise one or more incisors, one or more canines, and one or more premolars, and wherein the tongue guide (14) extends away from the lingual side of the one or more incisors, one or more canines, and one or more premolars.

8. The appliance (10) of claim 4, wherein the teeth comprise one or more incisors, one or more canines, one or more premolars, and one or more molars and wherein the tongue guide (14) extends away from the lingual side of the incisors, canines, premolars, and molars.

9. The appliance (10) of claim 4, wherein the tongue guide (14) defines a concave region into which a frenulum of the wearer is received.

10. The appliance (10) of claim 4, wherein tongue guide (14) comprises a first extension (14A) extending from the lingual side of the teeth toward a left side of a frenulum of the wearer and comprises a second extension (14A) extending from the lingual side of the teeth toward a right side of the frenulum.

11. The appliance (10) of claim 4, wherein the dental arch comprises a right half and a left half, wherein the tongue guide (14) extends from the left half to the right half.

12. The appliance (10) of claim 4, comprising two of the tongue guides (14).

13. The appliance (10) of claim 4, wherein the dental arch comprises a right half and a left half, wherein the tongue guide (14) extends from the left half to the right half.

14. The appliance (10) of claim 4, comprising two of the tongue guides (14), wherein the dental arch comprises a right half and a left half, and wherein one of the tongue guides (14) extends from the right half, and the other tongue guide (14) extends from the left half.

## Patentansprüche

1. Orale Vorrichtung (10), umfassend:
ein stützendes Schalenelement (12), aufweisend eine konkave Geometrie zum Aufnehmen mindestens eines Abschnitts eines Zahnbogens eines Trägers in der konkaven Geometrie; und
ein Zungenführungselement (14), das sich von dem stützenden Schalenelement (12) aus und in Kontakt mit einer Unterseite einer Zunge des Trägers erstreckt, wenn der Abschnitt des Zahnbogens in der konkaven Geometrie aufgenommen wird, wobei der Abschnitt des Bogens eine Bogengeometrie aufweist und wobei die konkave Geometrie über den Bogen passt und ein Negativ von mindestens einem Abschnitt der Bogengeometrie umfasst, und **dadurch gekennzeichnet, dass** das stützende Schalenelement (12) konfiguriert ist, um über das Zungenführungselement (14) zu passen.

2. Vorrichtung (10) nach Anspruch 1, wobei der Bogen Zähne umfasst, wobei jeder der Zähne eine einzigartige Zahngeometrie aufweist, und wobei die konkave Geometrie eine negative Geometrie umfasst, die der einzigartigen Zahngeometrie von mindestens einem der Zähne entspricht.

3. Vorrichtung (10) nach Anspruch 2, wobei das Zungenführungselement (14) eine Form umfasst, welche die Zunge nach oben in Richtung eines Gaumens des Trägers drückt und so positioniert ist, dass sie nach oben gedrückt wird.

4. Vorrichtung (10) nach Anspruch 3, wobei die Zähne eine linguale Seite aufweisen und das Zungenführungselement (14) sich von der lingualen Seite der Zähne weg erstreckt.

5. Vorrichtung (10) nach Anspruch 4, wobei die Zähne einen oder mehrere Schneidezähne umfassen, und wobei sich die Zungenführung (14) von der lingualen Seite der Schneidezähne weg erstreckt.

6. Vorrichtung (10) nach Anspruch 4, wobei die Zähne einen oder mehrere Schneidezähne und einen oder mehrere Eckzähne umfassen, und wobei sich die Zungenführung (14) von der lingualen Seite der Schneidezähne und Eckzähne weg erstreckt.

7. Vorrichtung (10) nach Anspruch 4, wobei die Zähne einen oder mehrere Schneidezähne, einen oder mehrere Eckzähne und einen oder mehrere Prämolaren umfassen, und wobei sich die Zungenführung (14) von der lingualen Seite des einen oder der mehreren Schneidezähne, des einen oder der mehreren Eckzähne und des einen oder der mehreren Prämolaren weg erstreckt.

8. Vorrichtung (10) nach Anspruch 4, wobei die Zähne einen oder mehrere Schneidezähne, einen oder mehrere Eckzähne, einen oder mehrere Prämolaren und einen oder mehrere Molaren umfassen, und wobei sich die Zungenführung (14) von der lingualen Seite der Schneidezähne, Eckzähne, Prämolaren und Molaren weg erstreckt.

9. Vorrichtung (10) nach Anspruch 4, wobei die Zungenführung (14) eine konkave Region definiert, in der ein Zungenbändchen des Trägers empfangen wird.

10. Vorrichtung (10) nach Anspruch 4, wobei die Zungenführung (14) eine erste Verlängerung (14A), die sich von der lingualen Seite der Zähne in Richtung einer linken Seite eines Zungenbändchens des Trägers erstreckt, und eine zweite Verlängerung (14A) umfasst, die sich von der lingualen Seite der Zähne in Richtung einer rechten Seite des Zungenbändchens erstreckt.

11. Vorrichtung (10) nach Anspruch 4, wobei der Zahnbogen eine rechte Hälfte und eine linke Hälfte umfasst, wobei sich die Zungenführung (14) von der linken Hälfte zur rechten Hälfte erstreckt.

12. Vorrichtung (10) nach Anspruch 4, umfassend zwei Zungenführungen (14).

13. Vorrichtung (10) nach Anspruch 4, wobei der Zahnbogen eine rechte Hälfte und eine linke Hälfte umfasst, wobei sich die Zungenführung (14) von der linken Hälfte zur rechten Hälfte erstreckt.

14. Vorrichtung (10) nach Anspruch 4, umfassend zwei Zungenführungen (14), wobei der Zahnbogen eine rechte Hälfte und eine linke Hälfte umfasst und wobei sich eine der Zungenführungen (14) von der rechten Hälfte aus erstreckt und sich die andere Zungenführung (14) von der linken Hälfte aus erstreckt.

## Revendications

1. Appareil buccal (10) comprenant :
un élément de coque de support (12) présentant une géométrie concave pour la réception d'au moins une partie d'une arcade dentaire d'un porteur dans la géométrie concave ; et
un élément de guidage de langue (14) s'étendant à partir dudit élément de coque de support (12) et en contact avec une face inférieure de la langue dudit porteur lorsque la partie de l'arcade dentaire est reçue dans la géométrie concave, dans lequel la partie de ladite arcade présente une géométrie d'arcade, et dans lequel la géométrie concave s'ajuste sur l'arcade et comprend un négatif d'au moins une partie de la géométrie d'arcade, et **caractérisé en ce que** l'élément de coque de support (12) est configuré pour s'ajuster sur l'élément de guidage de langue (14).

2. Appareil (10) selon la revendication 1, dans lequel l'arcade comprend des dents, chacune des dents présentant une géométrie de dent unique, et dans lequel la géométrie concave comprend une géométrie négative correspondant à la géométrie de dent unique d'au moins une desdites dents.

3. Appareil (10) selon la revendication 2, dans lequel l'élément de guidage de langue (14) comprend une forme qui va pousser, et est positionnée pour pousser, la langue vers le haut en direction d'un toit de la bouche du porteur.

4. Appareil (10) selon la revendication 3, dans lequel les dents présentent un côté lingual, et l'élément de guidage de langue (14) va s'étendre à partir du côté lingual des dents.

5. Appareil (10) selon la revendication 4, dans lequel les dents comprennent une ou plusieurs incisives, et dans lequel le guide de langue (14) s'étend à l'opposé du côté lingual des incisives.

6. Appareil (10) selon la revendication 4, dans lequel les dents comprennent une ou plusieurs incisives et une ou plusieurs canines, et dans lequel le guide de langue (14) s'étend à l'opposé du côté lingual des incisives et des canines.

7. Appareil (10) selon la revendication 4, dans lequel les dents comprennent une ou plusieurs incisives, une ou plusieurs canines, et une ou plusieurs prémolaires, et dans lequel le guide de langue (14) s'étend à l'opposé du côté lingual des une ou plusieurs incisives, des une ou plusieurs canines, et des une ou plusieurs prémolaires.

8. Appareil (10) selon la revendication 4, dans lequel les dents comprennent une ou plusieurs incisives, une ou plusieurs canines, une ou plusieurs prémolaires, et une ou plusieurs molaires et dans lequel le guide de langue (14) s'étend à l'opposé du côté lingual des incisives, des canines, des prémolaires, et des molaires.

9. Appareil (10) selon la revendication 4, dans lequel le guide de langue (14) définit une région concave dans laquelle un frein du porteur est reçu.

10. Appareil (10) selon la revendication 4, dans lequel le guide de langue (14) comprend une première extension (14A) s'étendant à partir du côté lingual des dents en direction d'un côté gauche d'un frein du porteur et comprend une seconde extension (14A) s'étendant à partir du côté lingual des dents en direction d'un côté droit du frein.

11. Appareil (10) selon la revendication 4, dans lequel l'arcade dentaire comprend une moitié droite et une moitié gauche, dans lequel le guide de langue (14) s'étend à partir de la moitié gauche vers la moitié droite.

12. Appareil (10) selon la revendication 4, comprenant deux des guides de langue (14).

13. Appareil (10) selon la revendication 4, dans lequel l'arcade dentaire comprend une moitié droite et une moitié gauche, dans lequel le guide de langue (14) s'étend à partir de la moitié gauche vers la moitié droite.

14. Appareil (10) selon la revendication 4, comprenant deux des guides de langue (14), dans lequel l'arcade dentaire comprend une moitié droite et une moitié gauche, et dans lequel un des guides de langue (14) s'étend à partir de la moitié droite, et l'autre guide de langue (14) s'étend à partir de la moitié gauche.
